(19) Europäisches Patentamt
European Patent Office
Office européen des brevets

(11) **EP 4 655 018 B1**

(12) **EUROPÄISCHE PATENTSCHRIFT**

(45) Veröffentlichungstag und Bekanntmachung des Hinweises auf die Patenterteilung:
**11.03.2026 Patentblatt 2026/11**

(21) Anmeldenummer: **25704211.9**

(22) Anmeldetag: **05.02.2025**

(51) Internationale Patentklassifikation (IPC):
***A61M 1/36*** *(2006.01)* ***A61M 1/16*** *(2006.01)*

(52) Gemeinsame Patentklassifikation (CPC):
**A61M 1/1609; A61M 1/165;** A61M 1/3656;
A61M 2205/3306; A61M 2205/3317; A61M 2205/52

(86) Internationale Anmeldenummer:
**PCT/EP2025/053008**

(87) Internationale Veröffentlichungsnummer:
**WO 2025/168657 (14.08.2025 Gazette 2025/33)**

(54) **EXTRAKORPORALE BLUTBEHANDLUNGSMASCHINE SOWIE COMPUTERPROGRAMM FÜR DIESE**

EXTRACORPOREAL BLOOD TREATMENT MACHINE AND COMPUTER PROGRAM THEREFOR

MACHINE DE TRAITEMENT EXTRACORPOREL DU SANG ET PROGRAMME INFORMATIQUE ASSOCIÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **06.02.2024 DE 102024103227**

(43) Veröffentlichungstag der Anmeldung:
**03.12.2025 Patentblatt 2025/49**

(73) Patentinhaber: **B. Braun Avitum AG**
**34212 Melsungen (DE)**

(72) Erfinder: **JANIK, Waldemar**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
**DE-A1- 102017 116 097 DE-A1- 102019 110 218**
**DE-A1- 102021 116 343**

## Beschreibung

## Technisches Gebiet

Die vorliegende Offenbarung betrifft eine extrakorporale Blutbehandlungsmaschine, insbesondere eine Dialysemaschine, für eine extrakorporale Blutbehandlung wie etwa eine Hämodialyse, eine Hämofiltration, eine Hämodiafiltration und/oder eine Ultrafiltration. Die Blutbehandlungsmaschine hat einen Dialysator mit einer semipermeablen Membran für einen Stoffaustausch zwischen einem Blut eines Patienten und einer Dialysierflüssigkeit, einen Dialysierflüssigkeitskreislauf mit einem (ersten) Verteiler-Abschnitt, welcher dafür angepasst ist, frische Dialysierflüssigkeit bzw. Dialyselösung bereitzustellen und verbrauchte Dialysierflüssigkeit bzw. Dialysat abzuführen, und einem (zweiten) Dialysator-Abschnitt, welcher mittels eines Dialysierflüssigkeitseingangs und eines Dialysierflüssigkeitsausgangs (des Dialysators) durch den Dialysator, das heißt durch eine Dialysierflüssigkeitsseite des Dialysators, verläuft, so dass die Dialysierflüssigkeit (als Fluid) durch den Dialysator förderbar ist, wobei die Blutbehandlungsmaschine dafür angepasst ist, zumindest zwischen einer Hauptschluss-Schaltung, in welcher der Verteiler-Abschnitt mit dem Dialysator-Abschnitt fluidtechnisch verbunden ist und frische Dialysierflüssigkeit durch den Dialysator fördert (oder diesen durchströmt), und einer Bypass-Schaltung, in welcher der Verteiler-Abschnitt von dem Dialysator-Abschnitt fluidtechnisch getrennt ist (und keine frische Dialysierflüssigkeit durch den Dialysator strömt), zu schalten. Insbesondere hat die Blutbehandlungsmaschine hierfür ein Fördermittel, wie etwa eine Dialysierflüssigkeitspumpe. Ferner hat die Blutbehandlungsmaschine einen extrakorporalen Blutkreislauf, welcher mittels eines Bluteingangs und -ausgangs (des Dialysators) durch den Dialysator, das heißt durch eine Blutseite des Dialysators, verläuft und dafür angepasst ist, insbesondere mittels eines Fördermittels, wie etwa einer Blutpumpe, Blut des Patienten durch den Dialysator zu fördern. Daneben betrifft die vorliegende Offenbarung ein Computerprogramm gemäß dem Oberbegriff des nebengeordneten Anspruchs.

## Technischer Hintergrund

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen und über einen extrakorporalen Blutkreislauf einem Dialysator für eine Blutbehandlung zugeführt. Dem Dialysator wird zudem eine Dialysierflüssigkeit über einen separaten Dialysierflüssigkeitskreislauf zugeführt. In dem Dialysator werden das Blut des Blutkreislaufs und die Dialysierflüssigkeit des Dialysierflüssigkeitskreislaufs über eine semipermeable Membran in Kontakt gebracht, so dass ein Stoffaustausch zwischen dem Blut und der Dialysierflüssigkeit stattfinden kann. So können bei der Dialysebehandlung niereninsuffizienter Patienten Schadstoffe aus dem Blut sowie auch überschüssiges Wasser, welches sich aufgrund eines zugrundeliegenden Nierenversagens im Körper ansammelt, entfernt werden. Das so gereinigte Blut wird anschließend über einen venösen Gefäßzugang an den Patienten wieder zurückgeführt.

Bei extrakorporalen Blutbehandlungsmaschinen kann nach aktuellem Stand der Technik von einem Hauptschluss in einen Bypass geschaltet werden. Während des geschalteten Bypasses wird die frische Dialysierflüssigkeit am Dialysator vorbeigeleitet und durchströmt diesen nicht. Durch geeignete Ventilstellungen in einem Dialysierflüssigkeitskreislauf der Blutbehandlungsmaschine wird dabei ein auf der Dialysierflüssigkeitsseite des Dialysators verbleibendes Dialysierflüssigkeitsfüllvolumen eingeschlossen.

Man kann auch sagen, dass der Dialysierflüssigkeitskreislauf einen ersten Verteiler-Abschnitt und einen zweiten Dialysator-Abschnitt (mit dem Dialysator) aufweist, die (insbesondere seriell) fluidtechnisch miteinander koppelbar bzw. verbindbar sowie auch abkoppelbar bzw. trennbar sind. In dem Hauptschluss bzw. der Hauptschluss-Schaltung liegt hierbei eine Fluidverbindung zwischen dem ersten Verteiler-Abschnitt und dem zweiten Dialysator-Abschnitt vor, die Dialysierflüssigkeit wird für eine Blutbehandlung von dem Verteiler-Abschnitt an den Dialysator-Abschnitt weitergeleitet, durchströmt den Dialysator und die verbrauchte Dialysierflüssigkeit bzw. das Dialysat wird anschließend wieder an den Verteiler-Abschnitt zurückgeführt. In dem Bypass, bzw. in der Bypass-Schaltung hingegen wird die Verbindung zwischen dem ersten Verteiler-Abschnitt und zweiten Dialysator-Abschnitt unterbrochen und die Dialysierflüssigkeit wird in dem Verteiler-Abschnitt, und damit vor dem Dialysator, fluidtechnisch umgeleitet. Es ergibt sich dadurch, dass die in dem Dialysator-Abschnitt vorliegende Dialysierflüssigkeit, und ganz besonders das in dem Dialysator eingesperrte Dialysierflüssigkeitsfüllvolumen, steht bzw. statisch ist und sich nicht bewegt. Es findet somit in dem Zustand der Bypass-Schaltung lediglich eine Diffusion zwischen dem eingesperrten Dialysierflüssigkeitsfüllvolumen einerseits und dem Blut des Blutkreislaufs andererseits statt, welche auf Grund der Sättigung besonders schnell an Dynamik verliert und mit Erreichen eines vollständigen, diffusiven Gleichgewichts abbricht.

Eine Schaltung der extrakorporalen Blutbehandlungsmaschine in einen Bypass kann dabei unterschiedliche Gründe haben. Beispielsweise kann bei einem Fehler einer Zusammensetzung der Dialysierflüssigkeit, insbesondere einer Ionenzusammensetzung, das medizinische Fachpersonal die Blutbehandlungsmaschine in den Bypass schalten (oder sogar die Blutbehandlungsmaschine automatisch selbst bei einem erkannten Fehler), um während dieser Zeit den Fehler zu beheben, während der Patient weiterhin am extrakorporalen Blutkreislauf angeschlossen bleibt. Auch kann in den Bypass geschaltet werden, wenn eine Änderung auf Seiten des Dialysats bzw. der Dialysierflüssigkeit dies erfordert, beispielsweise bei einem Tausch eines Beutels.

Darüber hinaus wird die Bypass-Schaltung insbesondere dafür genutzt, eine Kenngröße einer Leistungsfähigkeit des Dialysators in Bezug auf einen für die Blutbehandlung relevanten Bestandteil des Blutes treffen zu können. Gebräuchliche Kenngrößen sind beispielsweise die Dialysance oder die Clearance des Dialysators. Diese Kenngrößen hängen von dem Bluteingangswert und dem Dialysatausgangswert/ Dialysierflüssigkeitsausgangswert des relevanten Bestandteils ab. Während der Dialysatausgangswert vergleichsweise einfach gemessen/erfasst werden kann, ist der Aufwand zur Messung/Erfassung des Bluteingangswertes mit hohem Aufwand und mit erheblichen Unannehmlichkeiten für den Patienten verbunden.

Aus diesem Grunde schlägt die Druckschrift DE 197 34 992 C1 ein Verfahren zur rechnerischen Bestimmung des Bluteingangswertes als Basis zur Ermittlung der Dialysance vor. Dazu wird die Bypass-Schaltung so lange aufrechterhalten, bis sich das vollständige diffusive Gleichgewicht zwischen Blut- und Dialysierflüssigkeitsseite eingestellt hat. Aus dem dann erfassten Dialysatausgangswert des Bestandteils kann der Bluteingangswert und in Folge die Dialysance ermittelt werden. Nachteilig ist hier, dass die Bypass-Zeit, die zum Erreichen des vollständigen diffusiven Gleichgewichtes benötigt wird, relativ lang ist. Währenddessen kann die Blutbehandlung nicht weitergeführt werden, was die Therapiezeit erhöht und zudem der Gesundheit des Patienten unzuträglich ist.

Um das zu verbessern, schlägt die auf die Anmelderin zurückgehende Druckschrift DE 102017 116 097 A1 ein Verfahren für eine schnellere Bestimmung des bluteingangsseitigen Werts vor mit dem Ziel, dass das vollständige, diffusive Gleichgewicht während einer Bypass-Schaltung nicht mehr abgewartet werden muss. Dazu wird im Zuge der Blutbehandlung ein Skalierungsfaktor in Abhängigkeit eines Erfassungs-Signals des Bestandteils am Dialysatausgang ermittelt. Der Skalierungsfaktor setzt dabei eine Signaldifferenz nach langer Bypass-Zeit und vollständigem, diffusivem Gleichgewicht ins Verhältnis zu einer Signaldifferenz nach vorbestimmter, kürzerer Bypass-Zeit. Der ermittelte Skalierungsfaktor ermöglicht dann die Berechnung des Bluteingangswertes in Abhängigkeit des nach der vorbestimmten, kürzeren Bypass-Zeit erfassten Signals am Dialysatausgang/ Dialysierflüssigkeitsausgang.

Hierbei besteht ein Nachteil darin, dass der ermittelte Skalierungsfaktor allein für die vorbestimmte Bypass-Zeit gültig ist. Wird eine andere Bypass-Zeit erforderlich oder gewünscht, so muss das geschilderte Verfahren während der Blutbehandlung erneut angewendet werden, was zur erneuten Unterbrechung und damit Verlängerung der Blutbehandlung führt. In anderen Worten ausgedrückt, muss das Verfahren erneut angewendet werden, falls eine von bisherigen Bypass-Zeiten abweichende Bypass-Zeit erforderlich oder gewünscht sein sollte.

## Zusammenfassung der vorliegenden Offenbarung

Die Aufgabe der vorliegenden Offenbarung ist demgegenüber, die Nachteile aus dem Stand der Technik zu vermeiden oder zumindest zu mindern und insbesondere eine extrakorporale Blutbehandlungsmaschine, sowie ein Computerprogramm zur Verfügung zu stellen, welche(s) eine noch effizientere und sicherere Therapie einer extrakorporalen Blutbehandlung bereitstellt.

Die Aufgabe der vorliegenden Offenbarung wird hinsichtlich einer extrakorporalen Blutbehandlungsmaschine offenbarungsgemäß durch die Merkmale des Anspruchs 1 gelöst und hinsichtlich eines Computerprogramms offenbarungsgemäß durch die Merkmale des Anspruchs 14 gelöst.

Ein Grundgedanke der vorliegenden Offenbarung sieht vor, dass eine Blutbehandlungsmaschine dafür angepasst ist, dass während der Blutbehandlung zur Ermittlung eines Bestandteils im Blut an einem Bluteingang eines Dialysators der Blutbehandlungsmaschine, das heißt zur Ermittlung eines Bluteingangswert des Bestandteils, kein vorheriges Bestimmen eines Skalierungsfaktors nötig ist, weil dieser Skalierungsfaktor offenbarungsgemäß bereits in einem Speicher der Blutbehandlungsmaschine, beispielsweise in einem internen Speicher oder in einem externen Speicher, der beispielsweise mit einer Steuereinheit der Blutbehandlungsmaschine zumindest temporär kabelgebunden oder kabellos verbunden ist oder Bestandteil der Steuereinheit ist, in Form eines Kennfeldes oder in Form wenigstens einer Kennlinie hinterlegt und abrufbar ist, wobei das Kennfeld oder die wenigstens eine Kennlinie den Skalierungsfaktor zumindest in Abhängigkeit der Bypass-Zeit abbildet.

Mit anderen Worten wird eine extrakorporale Blutbehandlungsmaschine, insbesondere Dialysemaschine, beispielsweise Hämodialysemaschine, für eine extrakorporale Blutbehandlung von Blut eines Patienten vorgeschlagen, die aufweist:

einen Dialysator;
einen extrakorporalen Blutkreislauf, welcher über einen Bluteingang und Blutausgang des Dialysators durch diesen verläuft und dafür angepasst ist, Blut des Patienten durch den Dialysator zu fördern;
einen Dialysierflüssigkeitskreislauf mit fluidischen Schaltmitteln, mit einer Hauptschluss-Schaltung, in welcher der Dialysierflüssigkeitskreislauf über einen Dialysierflüssigkeitseingang und einen Dialysierflüssigkeitsausgang/ Dialysatausgang des Dialysators und durch den Dialysator geschaltet ist und dafür angepasst ist, frische Dialysierflüssigkeit bereitzustellen, durch den Dialysator zu fördern und verbrauchte Dialysierflüssigkeit bzw. Dialysat abzuführen, und mit einer Bypass-Schaltung, in welcher der Dialysierflüssigkeitskreislauf für die Dauer einer Bypass-Zeit am Dialysator vorbei geschaltet ist und dafür angepasst ist, ein Dialysierflüssigkeitsfüllvolumen in dem Dialysator einzusperren;

eine Erfassungseinheit, die dafür angepasst ist, an dem Dialysatausgang oder stromabwärts von dem Dialysatausgang einen Dialysatausgangswert eines Bestandteils im Dialysat zu erfassen, der mit einem Bluteingangswert des Bestandteils in dem Blut an dem Bluteingang korreliert, und ein Signal des Dialysatausgangswertes bereitzustellen, und;

eine Steuereinheit, die dafür angepasst ist, den Bluteingangswert in Abhängigkeit der zu Beginn der Bypasszeit und nach der Bypasszeit bereitgestellten Signale des Dialysatausgangswertes und eines von der Bypass-Zeit abhängigen, dialysatorspezifischen Faktors zu ermitteln.

Offenbarungsgemäß ist in einem Speicher der Blutbehandlungsmaschine, vorzugsweise in einem internen Speicher, der Bestandteil der Steuereinheit ist, oder in einem externen Speicher, der beispielsweise mit der Steuereinheit zumindest temporär kabelgebunden oder kabellos verbunden ist, wenigstens eine Kennlinie des dialysatorspezifischen Faktors, vorzugsweise ein Kennfeld des dialysatorspezifischen Faktors, insbesondere Skalierungsfaktors, zumindest in Abhängigkeit der Bypass-Zeit abgelegt und zur Ermittlung des Bluteingangswertes, insbesondere über die Steuereinheit, abrufbar. In anderen Worten ist der Skalierungsfaktor für ein von der Kennlinie oder dem Kennfeld abgedecktes Intervall der Bypass-Zeit in dem Speicher abgelegt und, insbesondere über die Steuereinheit, abrufbar.

Zur Ermittlung des Bluteingangswertes muss somit keine vorbestimmte Bypass-Zeit mehr eingehalten werden, um den gesuchten Bluteingangswert erfolgreich ermitteln zu können. Auf diese Weise können auch technisch oder anderweitig bedingte Bypass-Schaltungen, die beispielsweise in Abweichungen einer Zusammensetzung oder Temperatur der Dialysierflüssigkeit von ihrer jeweiligen Spezifikation begründet sind, zur Ermittlung des Bluteingangswertes genutzt werden. Ist/ sind diese Abweichungen behoben, kann unmittelbar in die Hauptschluss-Schaltung umgeschaltet werden. Die sich so ergebende - anstatt vorab gewählte - Bypass-Zeit dient dann der Ermittlung des Bluteingangswertes. Zur Ermittlung des Bluteingangswertes ist es somit offenbarungsgemäß möglich, auch Bypass-Zeiten, die sich als Notwendigkeit ergeben, zur Ermittlung des Bluteingangswertes nutzen zu können, ohne hierfür gesonderte, vorbestimmte Bypass-Zeiten vorsehen zu müssen, und/ oder die Bypass-Zeit frei, bevorzugt kurz, wählen zu können. Frei ist dabei im Rahmen dieser Offenbarung so zu verstehen, dass es sich um eine Bypass-Zeit handelt, die von der wenigstens einen Kennlinie/ dem Kennfeld abgebildet ist, oder in anderen Worten, dass der gewählten Bypass-Zeit in der wenigstens einen Kennlinie/ dem Kennfeld ein Wert des Faktors zugeordnet ist. Die experimentelle Ermittlung des Faktors, insbesondere Skalierungsfaktors, während der Blutbehandlung für eine gewünschte oder an einen situationsbedingten Bedarf angepasste Bypass-Zeit, oder gar wiederholte, experimentelle Ermittlungen dieses Faktors während der Blutbehandlung für unterschiedliche Bypass-Zeiten, wie es aus dem Stand der Technik bekannt ist, entfällt/ entfallen damit. Da die Dauer der Blutbehandlung unter anderem durch eine Anzahl und Dauer von Bypass-Zeiten bestimmt wird, kann somit eine Gesamt-Bypass-Zeit während der Blutbehandlung herabgesetzt werden, was dem Bedarf des Patienten, sich einer möglichst kurzen Blutbehandlung zu unterziehen, entgegenkommt. Die Bypass-Zeit kann so auf einfache Weise, angepasst an die Bedürfnisse des Patienten und die Anforderungen der Blutbehandlung, gewählt werden, was die Effizienz und Sicherheit der Blutbehandlung und für den Patienten erhöht.

Der Begriff des Dialysators betrifft eine Vorrichtung zur Blutreinigung, wie Hämodialyse, Hämofiltration und/oder Hämodiafiltration, insbesondere ein Dialysemodul, weiter bevorzugt ein Hohlfasermodul.

Der Begriff dialysespezifischer Faktor k meint vorliegend insbesondere einen Skalierungsfaktor zur Skalierung einer ersten Bypasszeit im Verhältnis zu einer zweiten Bypasszeit im Rahmen der Ermittlung eines gewünschten Bluteingangswertes CBI. Die erste Bypasszeit kann dabei vorzugsweise eine frei wählbare Bypasszeit meinen. Vorzugsweise kann die erste Bypasszeit eine zur zweiten Bypasszeit verkürzte Bypasszeit meinen. Vorzugsweise kann die zweite Bypasszeit eine Bypasszeit meinen, bis zu der sich ein vollständiges diffusives Gleichgewicht zwischen Blut- und Dialysierflüssigkeitsseite eingestellt hat. Der dialysespezifischer Faktor k ermöglicht eine Ermittlung eines Bluteingangswertes CBI für beliebige Bypasszeiten, sprich erste Bypasszeiten.

Der dialysespezifischer Faktor k kann durch folgende Gleichung ermittelt werden:

$$k = \frac{(CDOext - CDOpre)_{lange\ Bypasszeit}}{(CDOext - CDOpre)_{kurze\ Bypasszeit}}$$

wobei $CDO_{pre}$ für ein Signal des Dialysatausgangswertes CDO vor oder zu Beginn der Bypass-Zeit und $CDO_{ext}$ für einen Extremwert eines Signals des Dialysatausgangswertes CDO nach oder im Anschluss an die Bypass-Zeit stehen.

Das Signal kann beispielsweise eine Leitfähigkeit meinen. Das Signal kann beispielsweise eine Konzentration einer Substanz meinen. Die Substanz kann beispielsweise eine harnpflichte Substanz sein. Die Substanz kann beispielsweise eine lichtabsorbierende Substanz sein. Die Messung des Signals kann über eine Leitfähigkeitsmessung erfolgen. Die Messung des Signals kann über optische Messung erfolgen.

Vorteilhafte Ausführungsformen sind in den Unteransprüchen beansprucht und werden insbesondere nachfolgend erläutert.

Gemäß einer vorteilhaften Weiterbildung ist die wenigstens eine Kennlinie, vorzugsweise das Kennfeld, des dialysatorspezifischen Faktors zudem in Abhängigkeit eines Blutflusses in dem Speicher der Blutbehandlungsmaschine, vorzugsweise in der Steuereinheit, abgelegt und zur Ermittlung des Bluteingangswertes, insbesondere über die Steuereinheit, abrufbar. Dadurch ist eine Genauigkeit, mit der der Bluteingangswert ermittelt werden kann, erhöht.

Bevorzugt ist in dem Speicher, insbesondere in dem internen Speicher der Steuereinheit, eine Korrelation des Bluteingangswertes mit dem Dialysatausgangswert abgelegt, die von der Steuereinheit zur Ermittlung des Bluteingangswertes abrufbar ist. Der Bluteingangswert kann dabei in Abhängigkeit des erfassten Dialysatausgangswertes und des in Abhängigkeit der Bypass-Zeit und der wenigstens einen Kennlinie oder dem Kennfeld ermittelten Faktors ermittelt werden. Insbesondere lautet die Korrelation:

$$CBI = CDO_{\text{pre}} + k \cdot (CDO_{\text{ext}} - CDO_{\text{pre}}) \tag{1}$$

wobei $CDO_{\text{pre}}$ für das Signal des Dialysatausgangswertes CDO vor oder zu Beginn der Bypass-Zeit $t_{BYP}$ und $CDO_{\text{ext}}$ für einen Extremwert des Signals des Dialysatausgangswertes CDO nach oder im Anschluss an die Bypass-Zeit $t_{BYP}$ stehen.

Gemäß einer Weiterbildung umfassen die fluidischen Schaltmittel zumindest betätigbare Ventile und einen Bypass-Strömungspfad, die dafür angepasst sind, den Dialysierflüssigkeitskreislauf in die Hauptschluss-Schaltung und in die Bypass-Schaltung zu schalten.

Die fluidischen Schaltmittel sind vorzugsweise elektromagnetisch betätigbare, insbesondere von der Steuereinheit ansteuerbare, Sperrventile, insbesondere 2/2-Wege-Schaltventile.

Bevorzugt hat die extrakorporale Blutbehandlungsmaschine einen Dialysierflüssigkeitszulauf, der sich hin zu dem Dialysierflüssigkeitseingang erstreckt und über den der Dialysierflüssigkeitseingang mit einer Dialysierflüssigkeitsquelle, insbesondere mit einer Mischstation der Dialysierflüssigkeit bzw. Dialyselösung, fluidisch verbindbar oder verbunden ist. Zudem hat die extrakorporale Blutbehandlungsmaschine bevorzugt einen Dialysatablauf, über den der Dialysatausgang mit einer Dialysatsenke, insbesondere einem Reservoir für verbrauchte Dialysierflüssigkeit/ Dialysat, fluidisch verbindbar oder verbunden ist.

Der Bypass-Strömungspfad erstreckt sich vorzugsweise von dem Dialysierflüssigkeitszulauf zu dem Dialysatablauf, unter Umgehung des Dialysators, und insbesondere unter Umgehung des Dialysierflüssigkeitseingangs und -ausgangs.

Gemäß einer bevorzugten Weiterbildung ist je eines der Ventile zwischen einem Abzweig des Bypass-Strömungspfades von dem Dialysierflüssigkeitszulauf und dem Dialysierflüssigkeitseingang, sowie zwischen dem Dialysatausgang und einer Mündung des Bypass-Strömungspfades in den Dialysatablauf, sowie in dem Bypass-Strömungspfad angeordnet.

Das Kennfeld bildet in einfachster Ausgestaltung den Faktor allein in Abhängigkeit der Bypass-Zeit ab.

In weiterer Ausgestaltung bildet das Kennfeld den Faktor in Abhängigkeit der Bypass-Zeit und in Abhängigkeit eines Blutflusses im extrakorporalen Blutkreislauf ab.

In weiterer Ausgestaltung bildet das Kennfeld den Faktor in Abhängigkeit des Dialysierflüssigkeitsfüllvolumens, das heißt, in Abhängigkeit unterschiedlicher, spezifischer Dialysatoren, ab.

Besonders bevorzugt dient die Ermittlung des Bluteingangswertes der Ermittlung einer Kenngröße, die es erlaubt, eine Reinigungsleistung des Dialysators bezüglich eines im Blut zu reduzierenden Bestandteils zu bewerten. Eine hierfür gebräuchliche Kenngröße ist insbesondere eine Dialysance oder eine Clearance.

Gemäß einer bevorzugten Weiterbildung ist die Steuereinheit dafür angepasst, in Abhängigkeit des ermittelten Bestandteils im Blut am Bluteingang, und insbesondere in Abhängigkeit von Signalen des Blutausgangswertes vor und nach der Bypass-Zeit, einen Istwert wenigstens einer Kenngröße des Dialysators, insbesondere einer Dialysance und/oder einer Clearance, zu ermitteln.

Die Dialysance D ergibt sich bevorzugt zu:

$$D = -Q_{\text{D}} \frac{CDI_{\text{pre}} - CDO_{\text{pre}}}{CBI - CDI_{\text{pre}}} \tag{2}$$

mit dem Dialysatfluss $Q_{\text{D}}$ durch den Dialysator, einem Signal des Dialysierflüssigkeitseingangswertes/ Dialyselösungseingangswertes $CDI_{\text{pre}}$ am Dialysierflüssigkeitseingang vor oder zu Beginn der Bypass-Zeit $t_{BYP}$, dem Signal des Dialysatausgangswertes $CDO_{\text{pre}}$ vor oder zu Beginn der Bypass-Zeit $t_{BYP}$ und dem Extremwert des Signals des Dialysatausgangswertes $CDO_{\text{ext}}$ nach oder im Anschluss an die Bypass-Zeit $t_{BYP}$.

Die oben genannte Dialysance ist eine stoffabhängige Kenngröße. Das soll heißen, dass sie herangezogen wird, wenn es um einen Bestandteil im Blut geht, der auch in frischer Dialysierflüssigkeit vorgesehen ist.

Wird hingegen ein Bestandteil betrachtet, der in der frischen Dialysierflüssigkeit nicht vorkommt, so gilt $CDI_{pre}$ = 0, und man erhält die Clearance K und obige Gleichung (2) vereinfacht sich dann zu:

$$K = Q_D \frac{CDO_{pre}}{CBI} \tag{3}$$

Sowohl die Dialysance als auch die Clearance ist vom Dialysatfluss $Q_D$ abhängig, der gemäß einer bevorzugten Weiterbildung erfasst und/oder ermittelt ist.

Zu diesem Zweck weist die Blutbehandlungsmaschine gemäß einer Weiterbildung wenigstens eine Erfassungs- und/oder Ermittlungsvorrichtung zur Erfassung und/oder Ermittlung des Dialysatflusses $Q_D$ auf. Vorzugsweise ist diese Erfassungs- und/oder Ermittlungsvorrichtung in dem Dialysatablauf, vorzugsweise stromabwärts des Bypass-Strömungspfades, angeordnet.

Ist die Dialysance die zu ermittelnde Kenngröße, so wird zur Erfassung des Dialysatausgangswertes vorzugsweise eine Leitfähigkeit am Dialysatausgang erfasst. Dann hat oder ist die Erfassungseinheit an dem Dialysatausgang oder stromabwärts von diesem, die der Erfassung des Dialysatausgangswertes des Bestandteils im Dialysat am Dialysatausgang dient, gemäß der Offenbarung vorzugsweise eine Leitfähigkeits-Erfassungseinheit, die vorzugsweise temperaturkompensiert ist.

Zur Bestimmung der Clearance wird hingegen vorzugsweise eine Absorptionseigenschaft der verbrauchten Dialysierflüssigkeit/ des Dialysats herangezogen, insbesondere eine Extinktion, weshalb die Erfassungseinheit an dem Dialysatausgang oder stromabwärts von diesem, die der Erfassung des Dialysatausgangswertes des Bestandteils im Dialysat am Dialysatausgang dient, gemäß der Offenbarung vorzugsweise eine Absorptions-Erfassungseinheit hat oder ist.

Gemäß einer bevorzugten Weiterbildung ist die Steuereinheit der Blutbehandlungsmaschine dafür angepasst, ein Harnstoffverteilungsvolumen V des Patienten mittels einer in dem Speicher, insbesondere in dem internen Speicher der Steuereinheit, abgelegten und von der Steuereinheit aufrufbaren Korrelation abzuschätzen und in Abhängigkeit dieses abgeschätzten Harnstoffverteilungsvolumens V, der ermittelten Kenngröße, insbesondere der Clearance K, sowie einer, insbesondere in Minuten, ermittelten oder erfassten Blutbehandlungs- oder Dialysedauer t, eine Dialyseeffektivität Kt/V zu ermitteln.

Das Harnstoffverteilungsvolumen V ist insbesondere als Watson-Formel abgelegt, in die ein Körpergewicht, ein Alter, eine Größe und ein Geschlecht des Patienten eingehen. Sie lautet, jeweils in Abhängigkeit des Geschlechtes:

$$\text{Männlich: } V = 2{,}447 - 0{,}09516 \times \text{Alter} + 0{,}1074 \times \text{Größe} + 0{,}3362 \times \text{Gewicht} \tag{4}$$

$$\text{Weiblich: } V = -2{,}097 + 0{,}1069 \times \text{Größe} + 0{,}2466 \times \text{Gewicht} \tag{5}$$

Gemäß einer Weiterbildung der Blutbehandlungsmaschine ist die Steuereinheit dafür angepasst, in Abhängigkeit der ermittelten Dialyseeffektivität Kt/V eine äquilibrierte Dialyseeffektivität eKt/V zu ermitteln, durch welche ergänzend ein Effekt eines Harnstoff-Rebounds berücksichtigt wird. Hierfür ist in dem Speicher, insbesondere in dem internen Speicher der Steuereinheit, vorzugsweise folgende Gleichung abgelegt und von der Steuereinheit aufrufbar:

$$eKt/V = Kt/V - (0{,}6 \times Kt/V/T) + 0{,}03 \tag{6}$$

mit T der Dialysedauer in Stunden.

Gemäß einer bevorzugten Weiterbildung ist in dem Speicher, insbesondere in dem internen Speicher der Steuereinheit, ein Sollwert der wenigstens einen Kenngröße, insbesondere der Dialysance D und/oder Clearance K, abgelegt, und die Steuereinheit ist dafür angepasst, diesen aufzurufen und fortlaufend oder zeitaktuell eine Abweichung des Istwertes von dem Sollwert der wenigstens einen Kenngröße zu ermitteln, und insbesondere die ermittelte Abweichung bezüglich der Überschreitung einer Grenze, vorzugsweise zeitaktuell, zu überwachen.

In Abhängigkeit der Abweichung des Istwertes von dem Sollwert der wenigstens einen Kenngröße kann, insbesondere bei Überschreitung der Grenze, auf eine Leistungsminderung oder eine verringerte Effektivität oder Effizienz des Dialysators geschlossen werden.

Eine Leistungsminderung oder verringerte Effektivität oder Effizienz des Dialysators kann in einer Faserveränderung am Dialysator selbst oder in einem Fehler im extrakorporalen Blutkreislauf begründet sein.

Gemäß einer Weiterbildung ist die Steuereinheit dafür angepasst, zumindest in Abhängigkeit der Abweichung des Istwertes von dem Sollwert der wenigstens einen Kenngröße auf eine Faserveränderung des Dialysators und/oder auf einen Fehler im extrakorporalen Blutkreislauf zu schließen.

Die Faserveränderung ist insbesondere eine Sekundärmembranbildung und/oder eine Verklottung an Fasern, Holfasern oder Kapillaren des Dialysators.

Der Begriff der Verklottung beschreibt im Rahmen der Offenbarung einen Umstand, dass sich in dem Dialysator Hohlfasern oder Kapillare zusetzen und damit eine zur Blutreinigung zur Verfügung stehende Fläche verringert ist. Die Verklottung kann vielfältige Ursachen haben, beispielsweise eine Thrombozytenanlagerung an die Kapillare, eine Koagelbildung, eine zu geringe Zugabe von Gerinnungshemmern oder ein chemisches Binden von Blutbestandteilen an die Kapillare, oder dergleichen.

Der Fehler im extrakorporalen Blutkreislauf zeigt sich insbesondere in Form einer Zugangs-Rezirkulation. Eine Zugangs-Rezirkulation liegt vor, wenn der im extrakorporalen Blutkreislauf geförderte Blutfluss größer ist, als der Blutfluss am Zugang des Patienten. Durch die Zugangs-Rezirkulation wird ein Teil des bereits im Dialysator gereinigten Blutes erneut angesaugt, zum Bluteingang gefördert und in Folge erneut durch den Dialysator geleitet, was in der Konsequenz zur Abnahme der Dialysance oder Clearance, beziehungsweise zur bereits erwähnten Abweichung von deren jeweiligem Sollwert führt. Als Ursache der Zugangs-Rezirkulation kommt insbesondere eine Stenose in einem Gefäß des Patienten und eine suboptimale Punktierung in Frage.

Um die mögliche Faserveränderung am Dialysator von dem Fehler im extrakorporalen Blutkreislauf unterscheiden zu können, eignet sich insbesondere eine Erfassung und zeitliche Verfolgung des Bluteingangs-Drucks am Bluteingang des Dialysators. Alternativ oder ergänzend kann ein Transmembrandruck hierfür betrachtet werden, da ein Anstieg des Transmembrandrucks ebenfalls auf eine Faserveränderung, insbesondere Verklottung oder Sekundärmembranbildung hindeuten kann.

Gemäß einer Weiterbildung ist daher in dem extrakorporalen Blutkreislauf stromaufwärts des Bluteingangs eine Druckerfassungseinheit angeordnet, die dafür angepasst ist, den Bluteingangs-Druck zu erfassen und der Steuereinheit, insbesondere zeitaktuell, bereitzustellen, sodass diese ihn in dem Speicher als zeitlichen Verlauf ablegen kann. Offenbarungsgemäß ist die Steuereinheit zudem dafür angepasst, fortlaufend eine Abweichung, insbesondere einen Anstieg, des bereitgestellten Bluteingangs-Drucks relativ zu einem zu Anfang der Blutbehandlung bereitgestellten, und insbesondere in dem Speicher abgelegten, Bluteingangs-Druck zu ermitteln, und insbesondere diese ermittelte Abweichung bezüglich der Überschreitung einer Grenze, vorzugsweise zeitaktuell, zu überwachen.

Um die oben genannte Unterscheidung zwischen der möglichen Faserveränderung am Dialysator und dem Fehler im extrakorporalen Blutkreislauf treffen zu können, ist die Steuereinheit gemäß einer Weiterbildung dafür angepasst, dass, wenn sowohl die ermittelte Abweichung des Istwertes vom Sollwert der wenigstens einen Kenngröße, als auch der ermittelte Anstieg des bereitgestellten Bluteingangs-Drucks, hinreichend groß sind, das heißt größer oder gleich vorbestimmter Werte sind, einen Hinweis auf eine mögliche Faserveränderung und/oder Sekundärmembranbildung im Dialysator auszugeben.

Gemäß einer Weiterbildung ist die Steuereinheit dafür angepasst, einen Hinweis auf eine mögliche Zugangs-Rezirkulation in dem extrakorporalen Blutkreislauf auszugeben, wenn nur die ermittelte Abweichung des Istwertes vom Sollwert der wenigstens einen Kenngröße hinreichend groß, das heißt größer oder gleich einem vorbestimmten Wert, ist, und der Anstieg des Bluteingangs-Drucks hingegen nicht hinreichend groß, das heißt nicht größer oder gleich einem vorbestimmten Wert, ist.

Um die als möglich ermittelte/ausgegebene Rezirkulation zu bestätigen und insbesondere zu quantifizieren, ist die Steuereinheit gemäß einer Weiterbildung dafür angepasst, eine quantitative Rezirkulationsmessung durchzuführen. Die quantitative Rezirkulationsmessung kann automatisch über die Steuereinheit oder nach Ausgabe des Hinweises manuell eingeleitet werden. Über diese quantitative Rezirkulationsmessung können insbesondere ein Zustand des Zugangs/Shunts und insbesondere die korrekte Einstellung der Förderrate der Blutpumpe überwacht werden.

Gemäß einer Weiterbildung ist die Steuereinheit dafür angepasst, die Zugangs-Rezirkulation zu quantifizieren, indem in dem Speicher, insbesondere dem internen Speicher der Steuereinheit, eine Funktion der Zugangs-Rezirkulation als ein Berechnungsmodell, insbesondere als eine Lookup-Tabelle oder als charakteristische Kennlinie oder als charakteristisches Kennfeld, abgelegt und von der Steuereinheit auf-/abrufbar ist.

Insbesondere ist diese Funktion der Rezirkulation als Berechnungsmodell in Abhängigkeit folgender Größen, oder einer Auswahl davon, in dem Speicher abgelegt und von der Steuereinheit auf-/abrufbar: des Signals ($CDO_{pre,L}$) des Dialysatausgangswertes (CDO) zu Beginn einer Gleichgewichts-Bypasszeit ($t_{BYP,L}$), die so bemessen ist, dass sich ein vollständiges, diffusives Gleichgewicht einstellt; des Signals ($CDO_{ext,L}$) des Dialysatausgangswertes (CDO) nach der Gleichgewichts-Bypasszeit ($t_{BYP,L}$); des Signals ($CDO_{pre,K}$) des Dialysatausgangswertes (CDO) zu Beginn einer kürzeren Bypasszeit ($t_{BYP,K}$), die so bemessen ist, dass sich kein vollständiges, diffusives Gleichgewicht einstellt; des Signals ($CDO_{ext,K}$) des Dialysatausgangswertes (CDO) nach der kürzeren Bypasszeit ($t_{BYP,K}$); eines Blutflusses ($Q_B$) des extrakorporalen Blutkreislaufes; eines Dialysatflusses ($Q_D$) des Dialysierflüssigkeitskreislaufs; eines Delta-Quotienten (Vo), der eine Differenz aus dem Signal ($CDO_{pre,L}$) des Dialysatausgangswertes (CDO) zu Beginn der Gleichgewichts-Bypasszeit ($t_{BYP,L}$) und dem Signal ($CDO_{ext,L}$) des Dialysatausgangswertes (CDO) nach der Gleichgewichts-Bypasszeit ($t_{BYP,L}$) in Bezug setzt zu einer Differenz aus dem Signal ($CDO_{pre,K}$) des Dialysatausgangswertes (CDO) zu Beginn der kürzeren Bypasszeit ($t_{BYP,K}$) und dem Signal ($CDO_{ext,K}$) des Dialysatausgangswertes (CDO) nach der kürzeren Bypasszeit ($t_{BYP,K}$); eines Gesamtflächenintegral-Quotienten ($V_A$), durch den ein Gesamtflächenintegral unterhalb des Signals ($CDO_{ext,K}$) des Dialysatausgangswertes (CDO) nach der kürzeren Bypasszeit ($t_{BYP,K}$) in Bezug gesetzt ist zu einem Gesamtflächenintegral unterhalb des Signals ($CDO_{ext,L}$) des Dialysatausgangswertes (CDO) nach der Gleichgewichts-Bypasszeit ($t_{BYP,L}$); und/oder eines Teilflächenintegral-Quotienten ($V_{A,part}$), durch den ein Teilflächenintegral unterhalb des Signals ($CDO_{ext,K}$) des Dialysatausgangswertes (CDO) nach der kürzeren Bypasszeit ($t_{BYP,K}$) in Bezug gesetzt ist zu einem Teilflächenintegral unterhalb des Signals ($CDO_{ext,L}$) des Dialysatausgangswertes (CDO) nach der Gleichgewichts-Bypasszeit ($t_{BYP,L}$), wobei beide Teilflächenintegrale jeweils um das, insbesondere von der Bypass-Zeit-unabhängige Signal ($CDO_{pre,i}$) des Dialysatausgangswertes (CDO) zu Beginn der Bypasszeit ($t_{BYP,L}$, $t_{BYP,K}$), bereinigt sind.

Allgemein kann diese Funktion wie folgt in dem Speicher, insbesondere in dem internen Speicher der Steuereinheit, abgelegt und von der Steuereinheit auf-/abrufbar sein:

$$R = f\left(CDO_{pre,L}, CDO_{ext,L}, CDO_{pre,K}, CDO_{ext,K}, Q_B, Q_D, V_D, V_A, V_{A,part}\right) \tag{7}$$

Die als möglich ermittelte oder quantifizierte Zugangs-Rezirkulation, und/oder die als möglich ermittelte Faserveränderung des Dialysators kann/ können gemäß einer Weiterbildung der Blutbehandlungsmaschine einem Anwender, insbesondere dem medizinischen oder klinischen Personal, und/oder dem Patienten, auf einer Anzeigevorrichtung der extrakorporalen Blutbehandlungsmaschine oder auf einer Anzeigevorrichtung, die mit der extrakorporalen Blutbehandlungsmaschine kabellos oder kabelgebunden signalverbunden ist, dargestellt werden. Die Zugangs-Rezirkulation kann, wie oben bereits erwähnt, sowohl qualitativ (Vorhandensein der Zugangs-Rezirkulation als solche) als auch als quantitative Maßzahl oder als aktueller Rezirkulationswert, beispielsweise in %, ausgegeben werden. Die qualitative Ausgabe bedeutet dabei insbesondere, dass angezeigt wird, ob eine medizinisch relevante Zugangs-Rezirkulation vorliegt, was insbesondere bei einem Rezirkulationswert größergleich 15% der Fall ist. Eine halbquantitative Ausgabe kann insbesondere über ein Ampelfarben-System erfolgen.

Gemäß einer Weiterbildung ist die Steuereinheit dafür angepasst, den verwendeten, spezifischen Dialysator der Blutbehandlungsmaschine anhand eines Dialysierflüssigkeitsfüllvolumens automatisiert zu ermitteln.

Hierzu ist gemäß einer Weiterbildung die Steuereinheit bevorzugt dafür angepasst, eine Halbwertsbreite des nach Ablauf der Bypasszeit, das heißt nach Beendigung der Bypass-Schaltung, erfassten Signals des Dialysatausgangswertes zu ermitteln. Die Halbwertsbreite ist als Zeitspanne zwischen Zeitpunkten definiert, zu denen das Signal des Dialysatausgangswertes jeweils einen Wert aufweist, der eine Differenz zwischen einem Extremwert des Signals des Dialysatausgangswertes und dem zu Beginn der Bypass-Schaltung/ der Bypass-Zeit erfassten Signals des Dialysatausgangswertes halbiert.

Alternativ oder ergänzend ist die Steuereinheit dafür angepasst, eine Signaldauer des Dialysatausgangswertes zu ermitteln, welche sich von einem Ende der Bypass-Zeit bis zu einem Zeitpunkt erstreckt, zu dem das erfasste Signal des Dialysatausgangswertes gleich dem zu Beginn der Bypass-Zeit erfassten Signal des Dialysatausgangswertes ist.

Gemäß einer Weiterbildung ist die Steuereinheit zudem dafür angepasst, in Abhängigkeit der ermittelten Signaldauer und/ oder der ermittelten Halbwertsbreite, sowie des Dialysatflusses, das spezifische Dialysierflüssigkeitsfüllvolumen des verwendeten, spezifischen Dialysators zu ermitteln.

Da das Dialysierflüssigkeitsfüllvolumen eine spezifische Größe des verwendeten, spezifischen Dialysators oder Dialysator-Typs ist, kann aus dem ermittelten Dialysierflüssigkeitsfüllvolumen auf den verwendeten/ im Betrieb befindlichen, spezifischen Dialysator zurückgeschlossen werden.

Hierzu ist die Steuereinheit gemäß einer Weiterbildung dafür angepasst, in Abhängigkeit des ermittelten Dialysierflüssigkeitsfüllvolumens den verwendeten, spezifischen Dialysator zu ermitteln, wobei in dem Speicher, insbesondere dem internen Speicher der Steuereinheit, insbesondere in einer in dem Speicher abgelegten Lookup-Tabelle, spezifische Dialysatoren mit ihrem spezifischen Dialysierflüssigkeitsfüllvolumen abgelegt sind, und insbesondere die spezifischen Sollwerte für die Dialysance und/oder die Clearance abgelegt sind.

So ist die Steuereinheit dafür angepasst, bei initial unbekanntem Dialysator aus dem ermittelten Dialysatfluss $Q_D$ und der erfassten oder ermittelten Signaldauer und/ oder der erfassten oder ermittelten Halbwertsbreite den verwendeten/ im Betrieb befindlichen, spezifischen Dialysator und dessen spezifischen Sollwert(e) zu ermitteln.

Hinsichtlich eines Computerprogramms wird die Aufgabe der vorliegenden Offenbarung dadurch gelöst, dass dieses Computerprogramm Befehle umfasst, die bei einer Ausführung durch einen Computer diesen veranlassen ein computerimplementiertes Steuerverfahren für eine extrakorporale Blutbehandlungsmaschine (mit einem Dialysierflüssigkeitskreislauf, einem Blutkreislauf und einem Dialysator), insbesondere einer Blutbehandlungsmaschine gemäß der vorliegenden Offenbarung, auszuführen, wobei dieses Steuerverfahren die Schritte aufweist:

Schalten der Blutbehandlungsmaschine von einer Hauptschluss-Schaltung, in welcher ein Dialysierflüssigkeitseingang und ein Dialysierflüssigkeitsausgang/ Dialysatausgang des Dialysators geöffnet sind, sodass frische Dialysierflüssigkeit am Dialysierflüssigkeitseingang bereitgestellt ist, Dialysierflüssigkeit durch den Dialysator gefördert ist und verbrauchte Dialysierflüssigkeit bzw. Dialysat am Dialysatausgang abgeführt ist, in eine Bypass-Schaltung, in welcher der Dialysierflüssigkeitseingang und der Dialysatausgang gesperrt sind, und in welcher die frische Dialysierflüssigkeit am Dialysator vorbei geführt ist und ein Dialysierflüssigkeitsfüllvolumen in dem Dialysator eingesperrt ist;

Erfassen eines Dialysatausgangswertes eines Bestandteils im Dialysat, der mit einem Bluteingangswert des Bestandteils in dem Blut an dem Bluteingang korreliert, wobei das Erfassen an dem Dialysatausgang oder stromabwärts von diesem erfolgt, insbesondere zeitaktuell, über eine Erfassungseinheit;

Bereitstellen eines Signals des (zeitaktuell) erfassten Dialysatausgangswertes, insbesondere zeitaktuell, über die Erfassungseinheit;

Schalten der Blutbehandlungsmaschine von der Bypass-Schaltung in die Hauptschluss-Schaltung, sodass das eingesperrte Dialysierflüssigkeitsfüllvolumen, von dem insbesondere ein Dialysat-Bolus gebildet ist, am Dialysatausgang abgeführt ist; und in Folge

Bereitstellen einer Bypass-Zeit, für deren Dauer die Blutbehandlungsmaschine in die Bypass-Schaltung geschaltet war, über die Steuereinheit;

Abrufen eines Kennfeldes eines dialysatorspezifischen Faktors, welches in einem Speicher der Blutbehandlungsmaschine, vorzugsweise einem Speicher in der Steuereinheit, zumindest in Abhängigkeit der Bypass-Zeit abgelegt ist, über die Steuereinheit;

Ermitteln des dialysatorspezifischen Faktors in Abhängigkeit der bereitgestellten Bypass-Zeit aus dem Kennfeld, über die Steuereinheit, und

Ermitteln des Bluteingangswertes des Bestandteils in dem Blut an dem Bluteingang in Abhängigkeit der zu Beginn der bereitgestellten Bypasszeit, insbesondere vor der Bypass-Schaltung, und nach der bereitgestellten Bypasszeit, insbesondere nach der Bypass-Schaltung, bereitgestellten Signale des Dialysatausgangswertes und des ermittelten, dialysatorspezifischen Faktors, über die Steuereinheit.

Das Schalten von einer Hauptschluss-Schaltung in die Bypass-Schaltung, und zurück, von der Bypass-Schaltung in die Hauptschluss-Schaltung, erfolgt offenbarungsgemäß vorzugsweise automatisiert, insbesondere über die Steuereinheit.

Bevorzugt erfolgt das Schalten von der Hauptschluss-Schaltung in die Bypass-Schaltung, wenn von der Steuereinheit eine Abweichung einer Betriebsgröße der extrakorporalen Blutbehandlungsmaschine ermittelt wird, wie beispielsweise die Abweichung einer Konzentration oder Temperatur der Dialysierflüssigkeit und/ oder des Dialysats. Mit dem Schalten von der Hauptschluss-Schaltung in die Bypass-Schaltung erfolgt vorzugsweise eine Erfassung des Schaltzeitpunktes und/ oder ein Anstoßen einer Zeiterfassung zur Erfassung der Bypass-Zeit, für deren Dauer die Blutbehandlungsmaschine in die Bypass-Schaltung geschaltet ist.

Bevorzugt erfolgt das Schalten von der Bypass-Schaltung zurück in die Hauptschluss-Schaltung, wenn von der Steuereinheit die Abweichung als hinreichend klein oder ausgeregelt ermittelt wird. Mit dem Schalten von der Bypass-Schaltung in die Hauptschluss-Schaltung erfolgt vorzugsweise die Erfassung des Schaltzeitpunktes und/ oder ein Beenden der Zeiterfassung der Bypass-Zeit, für deren Dauer die Blutbehandlungsmaschine in die Bypass-Schaltung geschaltet war.

Vorzugsweise wird über die Steuereinheit in Abhängigkeit der beiden Schaltzeitpunkte die Bypass-Zeit, für deren Dauer die Blutbehandlungsmaschine in die Bypass-Schaltung geschaltet war, ermittelt.

Alternativ oder ergänzend kann Bedienpersonal das Schalten in die Bypass-Schaltung auslösen, beispielsweise durch Eingabe eines Befehls oder Betätigen eines gesonderten Betätigungselementes, insbesondere mit dem Ziel auf diese Weise die Ermittlung des Bluteingangswertes anzustoßen.

Vorzugsweise ist in der Steuereinheit für diesen Fall - der vom Bedienpersonal veranlassten Bypass-Schaltung - eine vorbestimmte Bypass-Zeit oder ein Satz von vorbestimmter Bypass-Zeiten hinterlegt. Vorzugsweise löst die o.g. Eingabe oder Betätigung die Zeiterfassung aus und die Blutbehandlungsmaschine wird bei Ablauf der vorbestimmten Bypass-Zeit über die Steuereinheit in die Hauptschluss-Schaltung zurückgeschaltet.

Im Anschluss an die beendete Bypass-Schaltung wird die aus den Schaltzeitpunkten ermittelte Bypass-Zeit und/ oder die mittels Zeiterfassung erfasste Bypass-Zeit, für deren Dauer die Blutbehandlungsmaschine in die Bypass-Schaltung geschaltet war, bereitstellbar/ abrufbar gespeichert, vorzugsweise in dem Speicher der Blutbehandlungsmaschine, insbesondere der Steuereinheit.

Bevorzugt weist das Steuerfahren ergänzend einen Schritt Ermitteln eines Istwertes wenigstens einer Kenngröße des Dialysators, insbesondere einer Dialysance und/oder einer Clearance, in Abhängigkeit des Bluteingangswertes in dem Blut an dem Bluteingang auf.

Bevorzugt weist das Steuerfahren ergänzend einen Schritt Erfassen und/oder Ermitteln eines Dialysierflüssigkeitsflusses oder Dialysatflusses auf.

Bevorzugt weist das Steuerfahren ergänzend Schritte Abschätzen eines Harnstoffverteilungsvolumens und Ermitteln einer Dialyseeffektivität in Abhängigkeit des Harnstoffverteilungsvolumens, der ermittelten Kenngröße, insbesondere der Clearance, sowie einer ermittelten oder erfassten Blutbehandlungs- oder Dialysedauer auf.

Bevorzugt weist das Steuerfahren ergänzend einen Schritt Ermitteln einer equilibrierten Dialyseeffektivität auf.

Bevorzugt weist das Steuerfahren ergänzend Schritte Ermitteln einer Abweichung eines Istwertes von einem Sollwert der wenigstens einen Kenngröße und Ermitteln einer Faserveränderung des Dialysators und/oder einer Zugangs-Rezirkulation im extrakorporalen Blutkreislauf in Abhängigkeit der ermittelten Abweichung der wenigstens einen Kenngröße auf.

Bevorzugt weist das Steuerfahren einen Schritt Ermitteln einer Abweichung, insbesondere eines Anstieges, eines Bluteingangs-Drucks relativ zu einem Bluteingangs-Druck zu Anfang der Blutbehandlung auf.

Bevorzugt weist das Steuerfahren einen Schritt Ausgeben eines Hinweises auf eine mögliche Faserveränderung des Dialysators auf, wenn sowohl die Abweichung des Istwertes vom Sollwert der wenigstens einen Kenngröße, als auch der Anstieg des Bluteingangs-Drucks als hinreichend groß, das heißt größer oder gleich vorbestimmter Werte, ermittelt sind.

Bevorzugt weist das Steuerfahren einen Schritt Ausgeben eines Hinweises auf eine mögliche Zugangs-Rezirkulation im extrakorporalen Blutkreislauf auf, wenn die Abweichung des Istwertes vom Sollwert der wenigstens einen Kenngröße als hinreichend groß, das heißt größer oder gleich einem vorbestimmten Wert, ermittelt ist, der Anstieg des Bluteingangs-Drucks hingegen nicht als hinreichend groß, das heißt nicht größer oder gleich einem vorbestimmten Wert, ermittelt ist.

Bevorzugt weist das Steuerfahren einen Schritt mit einer quantitativen Rezirkulationsmessung auf.

### Kurzbeschreibung der Figuren

Die Offenbarung wird nachfolgend anhand bevorzugter Ausführungsformen mit Hilfe von Figuren näher erläutert. Es zeigen:

Fig. 1      eine schematische Ansicht einer extrakorporalen Blutbehandlungsmaschine gemäß einer bevorzugten Ausführungsform;

Fig. 2      eine Regressions-Kennlinie eines dialysatorspezifischen Skalierungsfaktors in Abhängigkeit von einer Bypass-Zeit, ermittelt auf Basis von drei unterschiedlichen Blutflüssen;

Fig. 3      ein Kennfeld eines dialysatorspezifischen Skalierungsfaktors in Abhängigkeit von einer Bypass-Zeit und von unterschiedlichen Blutflüssen;

Fig. 4      Signale von Dialysatausgangswerten von Bestandteilen im Dialysat, erfasst an einem Dialysatausgang des Dialysators vor, während und nach der Bypass-Zeit;

Fig. 5      ein Flussdiagramm eines computerimplementierten Steuerverfahrens gemäß einer bevorzugten Ausführungsform; und

Fig. 6      ein Flussdiagramm des Steuerverfahren gemäß Figur 5 mit weiterführenden Schritten.

Die Figuren sind schematischer Natur und sollen nur dem Verständnis der Offenbarung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

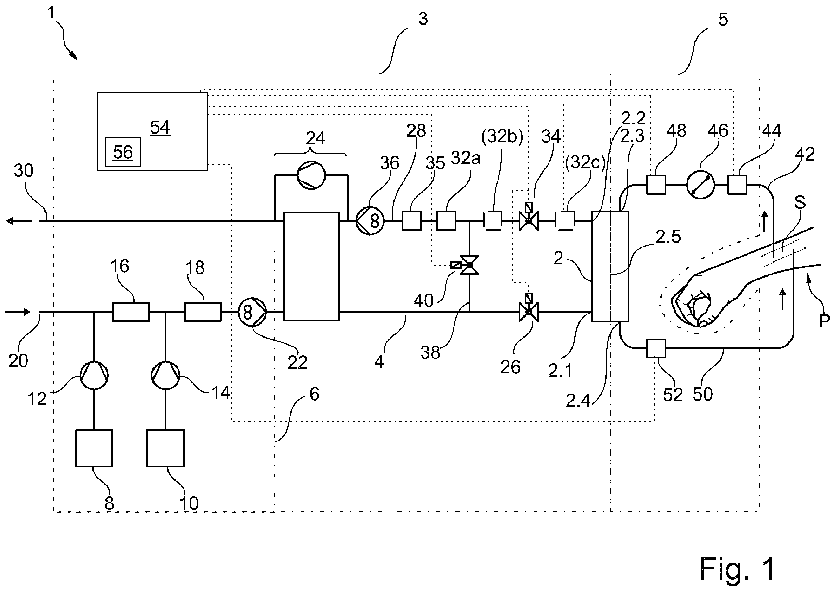

Figur 1 zeigt in einer schematischen Ansicht eine extrakorporale Blutbehandlungsmaschine 1 in Form einer Dialysemaschine für eine extrakorporale Blutbehandlung von Blut eines Patienten P gemäß einer bevorzugten Ausführungsform der vorliegenden Offenbarung.

Anhand Figur 1 werden im Folgenden eine Hauptschluss-Schaltung und eine Bypass-Schaltung eines Dialysierflüssigkeitskreislaufs 3 der Blutbehandlungsmaschine 1 erläutert, die dazu genutzt werden, die Konzentration eines bluteingangsseitigen Bestandteils des Blutes, einen sogenannten Bluteingangswert, ermitteln zu können. Die extrakorporale Blutbehandlungsmaschine 1 (nachfolgend nur Blutbehandlungsmaschine genannt) weist als zentrale Komponente einen Dialysator 2 mit einerseits je einem dialysierflüssigkeitsseitigen Dialysierflüssigkeitseingang 2.1 und Dialysatausgang 2.2 und andererseits je einem blutseitigen Bluteingang 2.3 und Blutausgang 2.4 auf. Innerhalb ist der Dialysator 2 mittels Hohlfasern einer semipermeablen Membran 2.5 in eine Dialysierflüssigkeitsseite und eine Blutseite aufgeteilt.

Der Dialysierflüssigkeitseingang 2.1 ist über einen Dialysierflüssigkeitszulauf 4 mit einer Dialysierflüssigkeitsbereitstellung 6 für frische Dialysierflüssigkeit fluidisch verbindbar, insbesondere verbunden.

Eine Dialysierflüssigkeitsbereitstellung 6 stellt kontinuierlich aus einem Permeat einem basischen Konzentrat und einem sauren Konzentrat Dialysierflüssigkeit her. Zugabemengen werden dabei von Messvorrichtungen kontrolliert. Dementsprechend hat die Dialysierflüssigkeitsbereitstellung 6 eine erste und zweite Quelle 8, 10 für basisches und für saures Konzentrat, eine erste und zweite Fördervorrichtung 12, 14 und stromabwärts der Fördervorrichtungen 12, 14 jeweils eine erste und zweite Messvorrichtung 16, 18. Zudem hat die Dialysierflüssigkeitsbereitstellung 6 einen Eingang 20 als Flüssigkeitszulauf. Die Dialysierflüssigkeitsbereitstellung 6 hat stromabwärts der zweiten Messvorrichtung 18 eine dritte Fördereinrichtung 22, über die die frische Dialysierflüssigkeit zu einer Bilanzierungsvorrichtung 24 gefördert wird. Ausgangsseitig ist die Bilanzierungsvorrichtung 24 über den Dialysierflüssigkeitszulauf 4 fluidisch mit dem Dialysierflüssigkeitseingang 2.1 des Dialysators 2 verbindbar, wobei im Dialysierflüssigkeitszulauf 4 ein Ventil 26 zum Absperren des Dialysierflüssigkeitseingangs 2.1 angeordnet ist.

Der Dialysatausgang 2.2 ist über einen Dialysatablauf 28 mit einem Entsorgungsausgang 30 für verbrauchte Dialysierflüssigkeit/ Dialysat fluidisch verbindbar, insbesondere verbunden. Im Dialysatablauf 28 sind, zwischen dem Dialysatausgang 2.2 und dem Entsorgungsausgang 30, fluidisch in Reihe angeordnet: ein betätigbares Ventil 34, vorzugsweise gleicher Bauart wie das Ventil 26, zum Absperren des Dialysatausgangs 2.2, eine Erfassungseinheit 32a zur Erfassung eines Bestandteils im Dialysat und eine vierte Fördervorrichtung 36, über die das Dialysat zur Bilanzierungsvorrichtung 24 und zum Entsorgungsausgang 30 für Dialysat gefördert wird. Die Bilanzierungsvorrichtung 24 sorgt dafür, dass ein gewünschtes Volumen an überschüssigem Wasser im Rahmen einer Ultrafiltration aus dem Patientenblut entzogen werden kann. Alternativ zur mit dem Bezugszeichen 32a gekennzeichneten Position im Dialysatablauf 24 kann diese Erfassungseinheit die Positionen 32b oder 32c aufweisen, d.h., stromabwärts des Ventils 34 und noch vor der Einmündung des Bypass-Strömungspfades 38 in den Dialysatablauf 24, oder zwischen dem Ventil 34 und dem Dialysatausgang 2.3. Stromaufwärts der vierten Fördervorrichtung 36 ist im Dialysatablauf 28 eine Druckerfassungseinheit 35 zur Erfassung eines Dialysatausgangs-Drucks vorgesehen.

Die Erfassungseinheit 32a zur Erfassung des Bestandteils im verbrauchten Dialysat kann in Form einer optischen Messvorrichtung mit einem Strahlungsemitter in Form einer LED und einem Photodetektor ausgeführt sein oder sie ist als eine, insbesondere temperaturkompensierte, Leitfähigkeits-Messvorrichtung ausgeführt.

Es können natürlich auch mehrere Bestandteile (A, B, ...) gleichzeitig erfasst werden. So könnte beispielsweise die Erfassungseinheit 32a eine Leitfähigkeit mit Bezug zu einem Bestandteil A erfassen und die Erfassungseinheit 32b eine Extinktion mit Bezug zu einem Bestandteil B.

Unabhängig von ihrer Ausführung ist die Erfassungseinheit 32a speziell dafür angepasst, permanent die Konzentration/ den Dialysatausgangswert CDO des Bestandteils in dem Dialysat zu erfassen und das entsprechendes Signal einer Steuerreinheit 54 der Blutbehandlungsmaschine 1 über eine Signalverbindung (gestrichelter Pfad) zeitaktuell bereitzustellen.

Ergänzend hat der Dialysierflüssigkeitskreislauf 3 einen Bypass-Strömungspfad 38, über den der Dialysierflüssigkeitszulauf 4 mit dem Dialysatablauf 28 fluidisch verbindbar ist. In dem Bypass-Strömungspfad 38 ist ein betätigbares Ventil 40, vorzugsweise gleicher Bauart wie die Ventile 26, 34, angeordnet, über das der Bypass-Strömungspfad 38 sperrbar ist.

Mit Hilfe der fluidischen Schaltmittel/ Ventile 26, 34 und 40 ist der Dialysierflüssigkeitskreislauf 3 über die Steuereinheit 54 in eine Hauptschluss-Schaltung schaltbar, in der über den Dialysierflüssigkeitszulauf 4 am Dialysierflüssigkeitseingang 2.1 frische Dialysierflüssigkeit bereitgestellt wird und durch den Dialysator 2 zum Dialysatausgang 2. 2 gefördert wird.

In der Hauptschluss-Schaltung sind die Ventile 26 und 34 des Dialysierflüssigkeitszulaufs 4 und des Dialysatablaufs 28 über die Steuereinheit 54 in ihre Offenstellung betätigt, während das Ventil 40 im Bypass-Strömungspfad 38 in seine Schließstellung betätigt ist, sodass der Bypass-Strömungspfad 38 gesperrt ist.

Des Weiteren ist der Dialysierflüssigkeitskreislauf 3 mittels der fluidischen Schaltmittel/ Ventile 26, 34 und 40 in eine Bypass-Schaltung schaltbar, in der jeweils der Dialysierflüssigkeitszulauf 4 vom Dialysierflüssigkeitseingang 2.1 und der Dialysatablauf 24 vom Dialysatausgang 2.2 fluidisch getrennt ist, während der Dialysierflüssigkeitszulauf 4 über den Bypass-Strömungspfad 38 mit dem Dialysatablauf 24 fluidisch verbunden ist. So strömt die frische Dialysierflüssigkeit nicht durch den Dialysator 2, sondern vom Dialysierflüssigkeitszulauf 4 - unter Umgehung des Dialysators 2 - über den geöffneten Bypass-Strömungspfad 38 direkt dem Dialysatablauf 28 zu. In der Bypass-Schaltung ist die Schaltung der Ventile 26, 34, 40 invers zur Hauptschluss-Schaltung, d.h. die Ventile 26 und 34 des Dialysierflüssigkeitszulaufs 4 und des Dialysatablaufs 28 sind über die Steuereinheit 54 in ihre Sperrstellung betätigt, während das Ventil 40 im Bypass-Strömungspfad 38 in seine Offenstellung betätigt ist.

Auf der Seite des Blutes ist ein extrakorporaler Blutkreislauf 5 vorgesehen, welcher über einen arteriellen Schlauchabschnitt 42 dem Patienten P Blut entnimmt und über den Bluteingang 2.3 dem Dialysator 2 zuführt. In dem arteriellen Schlauchabschnitt 42 sind in Strömungsrichtung ein arterieller Drucksensor 44, eine Blutpumpe 46 und ein Bluteingangs-Druck-Sensor 48 angeordnet. Nachdem im extrakorporalen Blutkreislauf 5 das Blut des Patienten P durch die Blutseite des Dialysators 2 geleitet wurde, wird es an dessen Blutausgang 2.4 entnommen und über einen venösen Schlauchabschnitt 50 dem Shunt S zugeführt. In dem venösen Schlauchabschnitt 50 ist ein Blutausgangs-Druck-Sensor 52 angeordnet.

Mit bluteingangsseitig, blutausgangsseitig und im Dialysatablauf 28 angeordneten Druckerfassungs-Sensoren/-Einheiten 48, 52 und 35 können mögliche Faserveränderungen, insbesondere eine Sekundärmembranbildung und/oder eine Verklottung, im Dialysator 2 auf Basis des sogenannten Transmembrandrucks TMP ermittelt werden. Ein Anstieg des Transmembrandrucks deutet dabei auf eine Verklottung oder eine Sekundärmembranbildung hin. Der Transmembrandruck kann gemäß folgender Gleichung berechnet werden:

$$TMP = (PBE + PV - 22 \text{ mmHg})/2 - (PDA - 16 \text{ mmHg}) \tag{8}$$

Mit PBE dem Bluteingangs-Druck am Bluteingang 2.3 des Dialysators 2, der vom dortigen Druck-Sensor 48 erfasst wird, PV dem venösen Druck am Blutausgang 2.4 des Dialysators 2, der vom dortigen Druck-Sensor 52 erfasst wird und PDA dem Dialysatausgangs-Druck im Dialysatablauf 28, der von der dortigen Druckerfassungseinheit 35 erfasst wird.

Das Blut wird in dem Dialysator 2 im Gegenstromverfahren zum Dialysat von harnpflichtigen Bestandteilen und überschüssigem Wasser befreit und hiernach gereinigt dem Patienten P zurückgegeben/ zurückgeführt.

Die Steuereinheit 54 ist mit den Ventilen 26, 34 und 40 signalverbunden, sodass sie diese in die vorbeschriebene Hauptschluss-Schaltung und Bypass-Schaltung ansteuern/betätigen kann. Zudem ist die Steuereinheit 54 mit den genannten Druckerfassungseinheiten 44, 48, 52, sowie Fördervorrichtungen 12, 14, 22, 36, Messvorrichtungen 16, 18, der Bilanziervorrichtung 24 und der Blutpumpe 46 signalverbunden, wobei aus Gründen der Übersichtlichkeit auf die Darstellung der jeweiligen Signalverbindung verzichtet wurde.

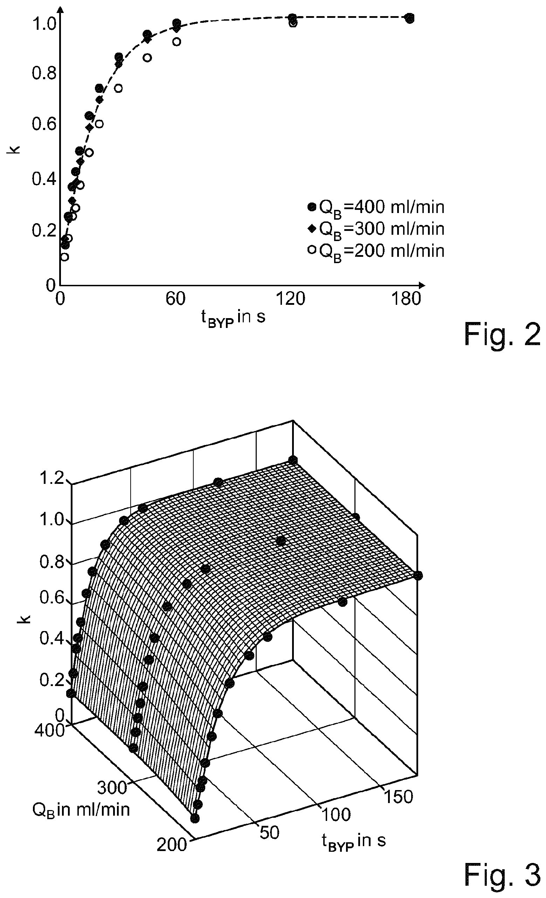

Es folgt die Beschreibung eines offenbarungsgemäßen Steuerverfahrens der Blutbehandlungsmaschine 1 gemäß Figur 1 unter Zuhilfenahme der Figuren 2, 3, 4 und 5.

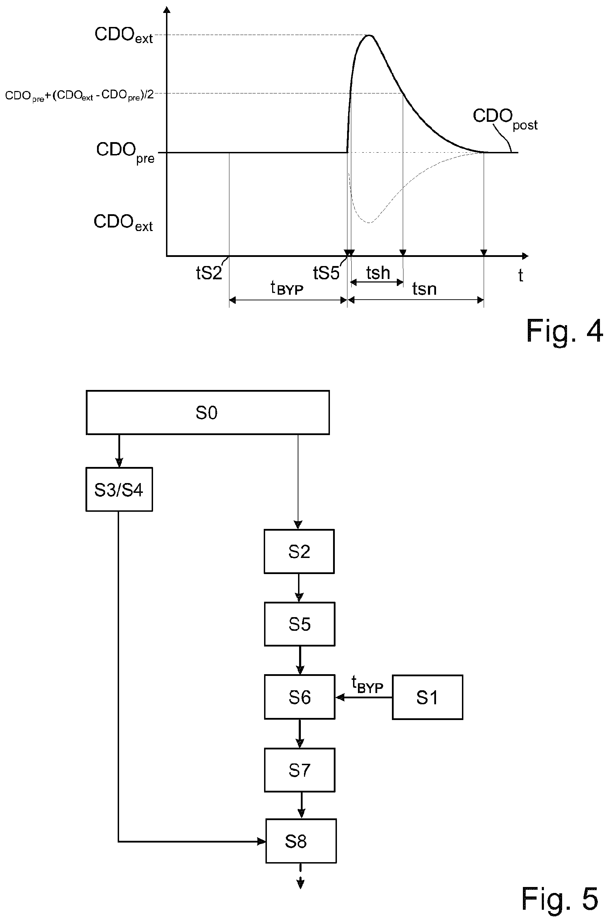

Einfach gesagt ist mittels der offenbarungsgemäßen Blutbehandlungsmaschine 1 gemäß Figur 1, beziehungsweise mittels dem offenbarungsgemäßen, vorzugsweise in der Steuereinheit 54 der Blutbehandlungsmaschine 1 zur Ausführung in einem Speicher 56 gespeicherten, Steuerverfahren gemäß Figur 5 eine Bypass-Schaltung mit nahezu beliebig wählbarer oder sich beliebig ergebender Bypass-Zeit $t_{BYP}$ möglich, um im Anschluss an die Bypass-Zeit $t_{BYP}$, basierend auf dem erfassten Signal eines Dialysatausgangswertes CDO eines Bestandteils am Dialysatausgang 2.2, einen Bluteingangswert CBI dieses Bestandteils am Bluteingang 2.3 des Dialysators 2 zu ermitteln.

Hierfür ist offenbarungsgemäß eine Kennlinie gemäß Figur 2 und/oder ein Kennfeld gemäß Figur 3 eines Skalierungsfaktors k in Abhängigkeit der Bypass-Zeit $t_{BYP}$ in dem Speicher 56 der Blutbehandlungsmaschine 1, insbesondere der Steuereinheit 54, abrufbar abgelegt. Alternativ kann der Speicher extern sein, wobei er mit der Steuereinheit 54 der Dialysemaschine 1 zumindest temporär, kabelbasiert oder kabellos, signalverbunden ist.

Mit Hilfe des Bluteingangswertes CBI kann im Anschluss eine Kenngröße des Dialysators, insbesondere eine Dialysance oder Clearance, ermittelt werden. Darüber hinaus kann mittels der Kenngröße überprüft werden, ob eine Zugangs-Rezirkulation vorliegt.

Figur 2 zeigt ein Ausführungsbeispiel einer in dem Speicher der Blutbehandlungsmaschine 1 abrufbar abgelegten Kennlinie/Regressionslinie des Skalierungsfaktors k eines spezifischen Dialysators 2, in Abhängigkeit der Bypass-Zeit $t_{BYP}$ (gestrichelte Kurve). Zur Ermittlung der Kennlinie/ Regressionslinie wurden mit dem spezifischen Dialysator 2 für unterschiedliche Bypass-Zeiten $t_{BYP}$ und für drei unterschiedliche Blutflüsse $Q_B$ (200, 300, 400ml/min) Labormessungen durchgeführt, aus denen die dargestellten Messpunkte für den Skalierungsfaktor k hervorgehen. Zwar ermöglicht die Kennlinie/ Regressionslinie gemäß Figur 2 die Bypass-Zeit $t_{BYP}$ während der Blutbehandlung zur Ermittlung des Bluteingangswertes frei wählen zu können und/ oder eine sich aus beispielsweise technischen Gründen ohnehin ergebende Bypass-Zeit $t_{BYP}$ zur Ermittlung des Bluteingangswertes nutzen zu können, allerdings zeigt Figur 2 auch, dass die Kennlinie/ Regressionskurve insbesondere bei mittleren Bypass-Zeiten $t_{BYP}$ und kleinerem Blutfluss $Q_B$ von den Messwerten abweicht.

Um hier für eine höhere Genauigkeit zu sorgen, wird gemäß einem Ausführungsbeispiel gemäß Figur 3 auf die Regressionskurve verzichtet und in dem Speicher 56 ist stattdessen ein Kennfeld des Skalierungsfaktors k in Abhängigkeit der Bypass-Zeit $t_{BYP}$ und zudem in Abhängigkeit des Blutflusses $Q_B$ abgelegt. Es ergibt sich gemäß Figur 3 eine Art Flächendarstellung des Skalierungsfaktors k über den Achsen der Bypass-Zeit $t_{BYP}$ und des Blutflusses $Q_B$.

Grundlegend wird der Bluteingangswert CBI in Abhängigkeit des Signals $CDO_{pre}$ des Dialysatausgangswertes vor der Bypass-Zeit $t_{BYP}$ und dem Extremwert des Signals $CDO_{ext}$ des Dialysatausgangswertes CDO im Anschluss an die Bypass-Zeit $t_{BYP}$ ermittelt. In diese Ermittlung geht der aus der Kennlinie gemäß Figur 2 oder dem Kennfeld gemäß Figur 3 für die konkret gewählte oder bereitgestellte Bypass-Zeit $t_{BYP}$ ermittelte Skalierungsfaktor k ein:

$$CBI = CDO_{\text{pre}} + k \cdot (CDO_{\text{ext}} - CDO_{\text{pre}}) \qquad (1)$$

Mögliche Signalverläufe des Dialysatausgangswertes CDO vor und im Anschluss an die Bypasszeit $t_{\text{BYP}}$, aus welchen die Signale $CDO_{\text{pre}}$ und $CDO_{\text{ext}}$ anschaulich hervorgehen, zeigt Figur 4. Für den Fall, dass eine Größe oder Komponente betrachtet wird, die im Blut niedriger sein kann als in der Dialysierflüssigkeit, insbesondere wenn beispielsweise das Signal des Dialysatausgangswertes CDO eine Leitfähigkeit als Maß für eine Ionenkonzentration oder -zusammensetzung ist, kann es sich bei dem nach der Bypass-Zeit $t_{\text{BYP}}$ auftretenden Extremwert $CDO_{\text{ext}}$ des Signals des Dialysatausgangswertes CDO um ein Minimum handeln, was in Figur 4 anhand des nach der Bypass-Zeit $t_{\text{BYP}}$ zunächst abfallenden, gestrichelten Kurvenverlaufs verdeutlicht werden soll. Ein Beispiel einer solchen Komponente ist beispielsweise Natrium, welches in der Bypass-Schaltung von der eingeschlossenen Dialysierflüssigkeit in den extrakorporalen Blutkreislauf übertritt. Im anderen Fall handelt es sich bei dem Extremwert $CDO_{\text{ext}}$ um ein Maximum, was anhand der durchgezogenen Kurve verdeutlicht wird.

Figur 4 zeigt darüber hinaus zwei unterschiedliche, mittels dem Signalverlauf des Dialysatausgangswertes CDO definierte, Zeitspannen $t_{\text{sn}}$ und $t_{\text{sh}}$, mit deren Hilfe ein Dialysierflüssigkeitsfüllvolumen des im Betrieb befindlichen Dialysators 2 ermittelt werden kann. Zum einen ist die Zeitspanne der Signaldauer $t_{\text{sn}}$ vom Ende der Bypass-Zeit $t_{\text{BYP}}$ bis zu dem Zeitpunkt, zu dem das Signal des Dialysatausgangswertes CDO wieder den Dialysatausgangswert $CDO_{\text{pre}}$ vor der Bypass-Zeit $t_{\text{BYP}}$ erreicht hat, definiert. Zum anderen ist die Zeitspanne der Halbwertsbreite $t_{\text{sh}}$ zwischen Zeitpunkten definiert, zu denen das Signal des Dialysatausgangswertes CDO auf der Hälfte zwischen dem Extremwert $CDO_{\text{ext}}$ und dem Wert $CDO_{\text{pre}}$ vor Beginn der Bypass-Zeit $t_{\text{BYP}}$ liegt. Genauer gesagt weist das Signal des Dialysatausgangswertes CDO zu diesen beiden Zeitpunkten folgenden Wert auf:

$$CDO = CDO_{\text{pre}} + (CDO_{\text{ext}} - CDO_{\text{pre}})/2 \qquad (8)$$

Um die Figur 4 nicht zu überfrachten, sind die beiden Zeitspannen $t_{\text{sn}}$ und $t_{\text{sh}}$ nur für den Signalverlauf mit dem Maximum (durchgezogene Kurve) dargestellt.

Figur 4 zeigt anschaulich, dass nach einer ausreichend langen Zeitspanne nach Beendigung der Bypass-Schaltung das Signal des Dialysatausgangswertes CDO einen Wert $CDO_{\text{post}}$ annimmt, der dem Wert $CDO_{\text{pre}}$ vor Beginn der Bypass-Zeit $t_{\text{BYP}}$ entspricht.

Aufbauend auf dem ermittelten Bluteingangswert CBI können in Folge insbesondere die Dialysance D oder die Clearance K des Dialysators 2 bezüglich des Bestandteils und ggf. weitere Kenngrößen des Dialysators 2 ermittelt werden.

Figur 5 zeigt ein Flussdiagramm eines computerimplementierten Steuerverfahrens für eine extrakorporale Blutbehandlungsmaschine 1 gemäß der vorliegenden Offenbarung. Das offenbarungsgemäße Steuerverfahren weist dabei zumindest die folgenden Schritte auf:

Starten S0 des Steuerverfahrens, insbesondere durch die Eingabe eines Anwenders an einer Bedienschnittstelle der Blutbehandlungsmaschine 1 oder über die Steuereinheit 54;

Schalten S2 der Blutbehandlungsmaschine 1 von der (oben beschriebenen) Hauptschluss-Schaltung in die Bypass-Schaltung;

Vorzugsweise permanentes Erfassen S3 des Dialysatausgangswertes CDO des Bestandteils im Dialysat stromabwärts des Dialysatausgangs 2.2 über die Erfassungseinheit 32a;

Vorzugsweise permanentes Bereitstellen S4 des Signals des erfassten Dialysatausgangswertes CDO, insbesondere zeitaktuell, über die Erfassungseinheit 32a;

Schalten S5 der Blutbehandlungsmaschine 1 von der Bypass-Schaltung in die Hauptschluss-Schaltung, sodass das eingesperrte Dialysierflüssigkeitsfüllvolumen am Dialysatausgang 2.2 abgeführt wird und die Erfassungseinheit 32a als ein Dialysat-Bolus passiert;

Bereitstellen S1 einer Bypass-Zeit $t_{\text{BYP}}$, das heißt einer Zeitdauer, während der die Blutbehandlungsmaschine 1 in die Bypass-Schaltung geschaltet war;

Abrufen S6 des Kennfeldes K des dialysatorspezifischen Faktors k aus dem Speicher 56;

Ermitteln S7 des dialysatorspezifischen Faktors k aus dem Kennfeld in Abhängigkeit der bereitgestellten Bypass-Zeit $t_{\text{BYP}}$; und

Ermitteln S8 des Bluteingangswertes CBI in Abhängigkeit der zu Beginn der bereitgestellten Bypasszeit $t_{\text{BYP}}$ und nach der bereitgestellten Bypasszeit $t_{\text{BYP}}$ von der Erfassungseinheit 32a bereitgestellten Signale $CDO_{\text{pre}}$, $CDO_{\text{ext}}$ des Dialysatausgangswertes CDO, sowie des ermittelten, dialysatorspezifischen Faktors k.

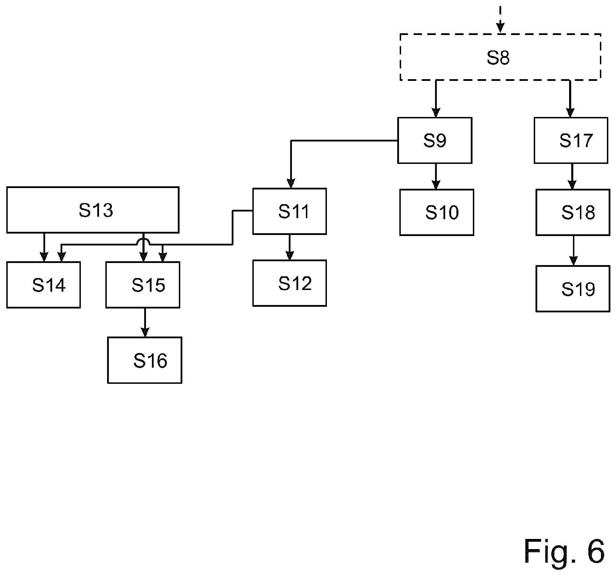

In Abhängigkeit des so ermittelten Bluteingangswertes CBI können darauf aufbauend gemäß Figur 6 weitere bevorzugte Schritte des Steuerverfahrens erfolgen, wie insbesondere:

Ermitteln S9 der wenigstens einen Kenngröße des Dialysators 2, insbesondere der Dialysance D und/oder der Clearance K, insbesondere deren Istwert, in Abhängigkeit des ermittelten Bluteingangswertes CBI, über die Steuereinheit 54;

Abschätzen S10 eines Harnstoffverteilungsvolumens V mittels einer in dem Speicher 56 abgelegten und aufrufbaren Korrelation und Ermitteln einer Dialyseeffektivität Kt/V in Abhängigkeit des Harnstoffverteilungsvolumens V, der zuvor ermittelten Kenngröße, insbesondere der Clearance K, sowie einer ermittelten oder erfassten Blutbehandlungs- oder Dialysedauer, über die Steuereinheit 54;

Ermitteln S11 einer Abweichung des Istwertes der wenigstens einen Kenngröße D, K von einem Sollwert, der in dem Speicher 56 abgelegt ist, über die Steuereinheit 54, insbesondere fortlaufend.

Ermitteln S12 einer möglichen Faserveränderung des Dialysators 2, insbesondere einer Sekundärmembranbildung und/oder einer Verklottung an Fasern des Dialysators 2, und/oder einer Zugangs-Rezirkulation im extrakorporalen Blutkreislauf 5, in Abhängigkeit der ermittelten Abweichung der wenigstens einen Kenngröße D, K, über die Steuereinheit 54.

Erfassen S13 des Bluteingangs-Drucks in dem extrakorporalen Blutkreislauf 5 stromaufwärts des Bluteingangs 2.3 und bereitstellen des Bluteingangs-Drucks, über die Druckerfassungseinheit 48 gemäß Figur 1, sowie fortlaufendes Ermitteln einer Abweichung, insbesondere eines Anstieges, des bereitgestellten Bluteingangs-Drucks relativ zu einem zu Anfang der Blutbehandlung bereitgestellten Bluteingangs-Druck, über die Steuereinheit 54.

Ausgeben S14 eines Hinweises auf eine mögliche Faserveränderung und/oder Sekundärmembranbildung im Dialysator 2, wenn sowohl die ermittelte Abweichung des Istwertes vom Sollwert der wenigstens einen Kenngröße D, K, als auch der ermittelte Anstieg des bereitgestellten Bluteingangs-Drucks hinreichend groß sind, das heißt, wenn sie jeweils oberhalb eines jeweiligen, vorbestimmten Grenzwertes liegen, über die Steuereinheit 54.

Ausgeben S15 eines Hinweises auf eine mögliche Zugangs-Rezirkulation R in dem extrakorporalen Blutkreislauf 5, wenn allein die ermittelte Abweichung des Istwertes vom Sollwert der wenigstens einen Kenngröße D, K hinreichend groß ist, das heißt, wenn sie oberhalb eines jeweiligen, vorbestimmten Grenzwertes liegt, über die Steuereinheit 54.

Quantifizieren S16 der Zugangs-Rezirkulation R, mittels der in dem Speicher 56 als ein Berechnungsmodell, insbesondere als eine Lookup-Tabelle oder als charakteristische Kennlinie oder charakteristisches Kennfeld, abgelegten und abrufbaren Funktion der Zugangs-Rezirkulation R, über die Steuereinheit 54.

Ermitteln S17 einer Signaldauer $t_{sn}$ des Dialysatausgangswertes CDO, die sich gemäß Figur 4 von einem Ende der Bypass-Zeit $t_{BYP}$ bis zu einem Zeitpunkt erstreckt, zu dem das Signal des Dialysatausgangswertes CDO wieder den vor der Bypass-Zeit $t_{BYP}$ erfassten Dialysatausgangswert $CDO_{pre}$ aufweist, und/ oder einer Halbwertsbreite $t_{sh}$ des Signals des Dialysatausgangswertes CDO nach einem Ende der Bypass-Zeit $t_{BYP}$, über die Steuereinheit 54.

Ermitteln S18 eines spezifischen Dialysierflüssigkeitsfüllvolumens $V_D$ des spezifischen Dialysators 2 in Abhängigkeit der Signaldauer $t_{sn}$ und/ oder der Halbwertsbreite $t_{sh}$, sowie eines Dialysatflusses $Q_D$, über die Steuereinheit 54.

Ermitteln S19 des spezifischen Dialysators 2 in Abhängigkeit des ermittelten Dialysierflüssigkeitsfüllvolumens $V_D$, insbesondere mittels einer in dem Speicher 56 abgelegten Lookup-Tabelle, in welcher spezifische Dialysatoren mit ihrem spezifischen Dialysatfüllvolumen $V_D$ abgelegt sind, und in der insbesondere dialysatorspezifische Sollwerte für die Dialysance D und/oder die Clearance K abgelegt sind. Über die Steuereinheit 54.

Bezugszeichenliste

1      Extrakorporale Blutbehandlungsmaschine
2      Dialysator
2.1    Dialysierflüssigkeitseingang
2.2    Dialysatausgang
2.3    Bluteingang
2.4    Blutausgang
2.5    Semipermeable Membran
3      Dialysierflüssigkeitskreislauf
4      Dialysierflüssigkeitszulauf
6      Dialysierflüssigkeitsbereitstellung
8      erste Quelle saures Konzentrat
10     zweite Quelle basisches Konzentrat
12     erste Fördervorrichtung
14     zweite Fördervorrichtung

EP 4 655 018 B1

| | |
|---|---|
| 16 | erste Messvorrichtung |
| 18 | zweite Messvorrichtung |
| 20 | Reinwassereingang |
| 22 | dritte Fördervorrichtung |
| 24 | Bilanziervorrichtung |
| 26 | erstes Ventil |
| 28 | Dialysatablauf |
| 30 | Entsorgungsausgang |
| 32a | Erfassungseinheit |
| 32b | Erfassungseinheit (optional) |
| 32c | Erfassungseinheit (optional) |
| 34 | zweites Ventil |
| 35 | Druckerfassungseinheit |
| 36 | vierte Fördervorrichtung |
| 38 | Bypass-Strömungspfad |
| 40 | drittes Ventil |
| 42 | arterieller Schlauchabschnitt |
| 44 | arterieller Drucksensor |
| 46 | Blutpumpe |
| 48 | Bluteingangs-Druck-Sensor |
| 50 | venöser Schlauchabschnitt |
| 52 | Blutausgangs-Druck-Sensor |
| 54 | Steuereinheit |
| 56 | Speicher |

| | |
|---|---|
| S0 | Start Steuerverfahren |
| S1 | Schritt Bereitstellen Bypass-Zeit |
| S2 | Schritt Schalten Hauptschluss in Bypass |
| S3 | Schritt Erfassen Dialysatausgangswert |
| S4 | Schritt Bereitstellen Signal Dialysatausgangswert |
| S5 | Schritt Schalten Bypass in Hauptschluss |
| S6 | Schritt Abrufen Kennfeld eines Faktors |
| S7 | Schritt Ermitteln Faktor |
| S8 | Schritt Ermitteln Bluteingangswert |
| S9 | Schritt Ermitteln Kenngröße |
| S10 | Schritt Abschätzen Harnstoffverteilungsvolumen |
| S11 | Schritt Ermitteln Abweichung Kenngröße |
| S12 | Schritt Ermitteln mögliche Faserveränderung |
| S13 | Schritt Erfassen Bluteingangs-Druck |
| S14 | Schritt Ausgeben Hinweis mögliche Faserveränderung |
| S15 | Schritt Ausgeben Hinweis mögliche Zugangs-Rezirkulation |
| S16 | Schritt Quantifizieren Zugangs-Rezirkulation |
| S17 | Schritt Ermitteln Signaldauer/ Halbwertsbreite |
| S18 | Schritt Ermitteln Dialysierflüssigkeitsfüllvolumen |
| S19 | Schritt Ermitteln Dialysator |

| | |
|---|---|
| P | Patient |
| S | Shunt |
| tS2 | Start Bypass-Zeit |
| tS5 | Ende Bypass-Zeit |
| $t_{BYP}$ | Bypass-Zeit |
| $t_{sn}$ | Signaldauer |
| $t_{sh}$ | Halbwertsbreite |
| CDO | Dialysatausgangswert |
| $CDO_{pre}$ | Dialysatausgangswert vor Bypass-Zeit |
| $CDO_{ext}$ | maximaler Dialysatausgangswert nach Bypass-Zeit |
| $CDO_{post}$ | abgeklungener Dialysatausgangswert |
| k | dialysatorspezifischer Skalierungsfaktor |

15

**Patentansprüche**

1. Extrakorporale Blutbehandlungsmaschine (1), insbesondere Dialysemaschine, für eine extrakorporale Blutbehandlung von Blut eines Patienten (P), aufweisend:

   - einen Dialysator (2),
   - einen extrakorporalen Blutkreislauf (5), welcher über einen Bluteingang (2.3) und einen Blutausgang (2.4) des Dialysators (2) durch diesen verläuft und dafür angepasst ist, Blut des Patienten (P) durch den Dialysator (2) zu fördern,
   - einen Dialysierflüssigkeitskreislauf (3) mit fluidischen Schaltmitteln (26, 34, 40), mit einer Hauptschluss-Schaltung, in welcher der Dialysierflüssigkeitskreislauf (3) über einen Dialysierflüssigkeitseingang (2.1) und einen Dialysatausgang (2.2) des Dialysators (2) durch den Dialysator (2) geschaltet ist und dafür angepasst ist, frische Dialysierflüssigkeit bereitzustellen, durch den Dialysator (2) zu fördern und verbrauchte Dialysierflüssigkeit bzw. Dialysat abzuführen, und einer Bypass-Schaltung, in welcher der Dialysierflüssigkeitskreislauf (3) für die Dauer einer Bypass-Zeit ($t_{BYP}$) am Dialysator (2) vorbei geschaltet ist und dafür angepasst ist, ein Dialysierflüssigkeitsfüllvolumen ($V_D$) in dem Dialysator (2) einzusperren,
   - eine Erfassungseinheit (32a, 32b, 32c), die dafür angepasst ist, an dem Dialysatausgang oder stromabwärts von dem Dialysatausgang (2.2) einen Dialysatausgangswert (CDO) eines Bestandteils im Dialysat zu erfassen, der mit einem Bluteingangswert (CBI) des Bestandteils in dem Blut an dem Bluteingang (2.3) korreliert, und ein Signal ($CDO_{pre}$, $CDO_{ext}$) des Dialysatausgangswertes (CDO) bereitzustellen, und
   - eine Steuereinheit (54), die dafür angepasst ist, den Bluteingangswert (CBI) in Abhängigkeit der zu Beginn der Bypasszeit ($t_{BYP}$) und nach der Bypasszeit ($t_{BYP}$) bereitgestellten Signale ($CDO_{pre}$, $CDO_{ext}$) des Dialysatausgangswertes (CDO) und eines von der Bypass-Zeit ($t_{BYP}$) abhängigen, dialysatorspezifischen Faktors (k) zu ermitteln,

   **dadurch gekennzeichnet, dass** in einem Speicher (56) der Blutbehandlungsmaschine (1), vorzugsweise in der Steuereinheit (54), wenigstens eine Kennlinie, vorzugsweise ein Kennfeld, des dialysatorspezifischen Faktors (k) zumindest in Abhängigkeit der Bypass-Zeit ($t_{BYP}$) abgelegt und zur Ermittlung des Bluteingangswertes (CBI) abrufbar ist.

2. Extrakorporale Blutbehandlungsmaschine (1) nach Anspruch 1, **dadurch gekennzeichnet, dass** in dem Speicher (56) der Blutbehandlungsmaschine (1), vorzugsweise in der Steuereinheit (54), die wenigstens eine Kennlinie, vorzugsweise das Kennfeld, des dialysatorspezifischen Faktors (k) in Abhängigkeit eines Blutflusses ($Q_B$) abgelegt und zur Ermittlung des Bluteingangswertes (CBI) abrufbar ist.

3. Extrakorporale Blutbehandlungsmaschine (1) nach Anspruch 1 oder 2, **dadurch gekennzeichnet, dass** die Steuereinheit (54) dafür angepasst ist, in Abhängigkeit des ermittelten Bestandteils im Blut am Bluteingang (CBI) einen Istwert wenigstens einer Kenngröße (D, K) des Dialysators (2), insbesondere einer Dialysance (D) und/oder einer Clearance (K), zu ermitteln.

4. Extrakorporale Blutbehandlungsmaschine (1) nach Anspruch 3, **dadurch gekennzeichnet, dass** die Steuereinheit (54) dafür angepasst ist, ein Harnstoffverteilungsvolumen (V) mittels einer in dem Speicher (56) abgelegten und aufrufbaren Korrelation abzuschätzen und in Abhängigkeit dieses abgeschätzten Harnstoffverteilungsvolumens (V), der ermittelten Kenngröße (K), insbesondere der Clearance (K), sowie einer, insbesondere in Minuten, ermittelten oder erfassten Blutbehandlungs- oder Dialysedauer (t) eine Dialyseeffektivität (Kt/V) zu ermitteln.

5. Extrakorporale Blutbehandlungsmaschine (1) nach Anspruch 3 oder 4, **dadurch gekennzeichnet, dass** in dem Speicher (56) ein Sollwert der wenigstens einen Kenngröße (D, K), insbesondere der Dialysance (D) und/oder Clearance (K), abgelegt ist, und die Steuereinheit (54) dafür angepasst ist, fortlaufend eine Abweichung des Istwertes vom Sollwert der wenigstens einen Kenngröße (D, K) zu ermitteln.

6. Extrakorporale Blutbehandlungsmaschine (1) nach Anspruch 5, **dadurch gekennzeichnet, dass** die Steuereinheit (54) dafür angepasst ist, zumindest in Abhängigkeit der ermittelten Abweichung der wenigstens einen Kenngröße (D, K) eine Faserveränderung des Dialysators (2), insbesondere eine Sekundärmembranbildung und/oder eine Verklottung an Fasern des Dialysators (2), und/oder eine Zugangs-Rezirkulation im extrakorporalen Blutkreislauf (5) zu ermitteln.

7. Extrakorporale Blutbehandlungsmaschine (1) nach einem der vorhergehenden Ansprüche, **dadurch gekennzeich-**

**net, dass** in dem extrakorporalen Blutkreislauf (5) stromaufwärts des Bluteingangs (2.3) eine Druckerfassungseinheit (48) angeordnet ist, die dafür angepasst ist, einen Bluteingangs-Druck zu erfassen und der Steuereinheit (54), insbesondere zeitaktuell, bereitzustellen, und dass die Steuereinheit (54) dafür angepasst ist, fortlaufend eine Abweichung, insbesondere einen Anstieg, des bereitgestellten Bluteingangs-Drucks relativ zu einem zu Anfang der Blutbehandlung bereitgestellten Bluteingangs-Druck zu ermitteln.

8. Extrakorporale Blutbehandlungsmaschine (1) zumindest nach Anspruch 5 und 7, **dadurch gekennzeichnet, dass** die Steuereinheit (54) dafür angepasst ist, dass, wenn sowohl die ermittelte Abweichung des Istwertes vom Sollwert der wenigstens einen Kenngröße (D, K), als auch der ermittelte Anstieg des bereitgestellten Bluteingangs-Drucks hinreichend groß ist, einen Hinweis auf eine mögliche Faserveränderung und/oder Sekundärmembranbildung im Dialysator (2) auszugeben.

9. Extrakorporale Blutbehandlungsmaschine (1) zumindest nach Anspruch 5 und 7, **dadurch gekennzeichnet, dass** die Steuereinheit (54) dafür angepasst ist, dass, wenn allein die ermittelte Abweichung des Istwertes vom Sollwert der wenigstens einen Kenngröße (D, K) hinreichend groß ist, einen Hinweis auf eine mögliche Zugangs-Rezirkulation in dem extrakorporalen Blutkreislauf (5) auszugeben.

10. Extrakorporale Blutbehandlungsmaschine (1) nach Anspruch 9, **dadurch gekennzeichnet, dass** die Steuereinheit (54) dafür angepasst ist, die Zugangs-Rezirkulation (R) zu quantifizieren, indem in dem Speicher (56) eine Funktion der Zugangs-Rezirkulation (R) als ein Berechnungsmodell, insbesondere als eine Lookup-Tabelle oder als charakteristische Kennlinie oder charakteristisches Kennfeld, abgelegt und von der Steuereinheit (54) abrufbar ist.

11. Extrakorporale Blutbehandlungsmaschine (1) nach einem der vorhergehenden Ansprüche, **dadurch gekennzeichnet, dass** die Steuereinheit (54) dafür angepasst ist, eine Signaldauer ($t_{sn}$) des Dialysatausgangswertes (CDO) zu ermitteln, die sich von einem Ende der Bypass-Zeit ($t_{BYP}$) bis zu einem Zeitpunkt erstreckt, zu dem das Signal des Dialysatausgangswertes (CDO) wieder den vor der Bypass-Zeit ($t_{BYP}$) erfassten Dialysatausgangswert ($CDO_{pre}$) aufweist, und/ oder dass die Steuereinheit (54) dafür angepasst ist, nach einem Ende der Bypass-Zeit ($t_{BYP}$) eine Halbwertsbreite ($t_{sh}$) des Signals des Dialysatausgangswertes (CDO) zu ermitteln.

12. Extrakorporale Blutbehandlungsmaschine (1) nach Anspruch 11, **dadurch gekennzeichnet, dass** die Steuereinheit (54) dafür angepasst ist, in Abhängigkeit der Signaldauer ($t_{sn}$) oder der Halbwertsbreite ($t_{sh}$), sowie eines Dialysatflusses ($Q_D$) ein spezifisches Dialysierflüssigkeitsfüllvolumen ($V_D$) des spezifischen Dialysators (2) zu ermitteln.

13. Extrakorporale Blutbehandlungsmaschine (1) nach Anspruch 12, **dadurch gekennzeichnet, dass** die Steuereinheit (54) dafür angepasst ist, in Abhängigkeit des ermittelten Dialysierflüssigkeitsfüllvolumens ($V_D$) den spezifischen Dialysator (2) zu ermitteln, wobei in dem Speicher (56), insbesondere in einem in dem Speicher (56) abgelegten Lookup-Tabelle, spezifische Dialysatoren mit ihrem spezifischen Dialysatfüllvolumen (Vo) abgelegt sind, und insbesondere dialysatorspezifische Sollwerte für die Dialysance (D) und/oder die Clearance (K) abgelegt sind.

14. Computerprogramm, umfassend Befehle, die bei einer Ausführung durch die Steuereinheit einer extrakorporalen Blutbehandlungsmaschine nach einem der vorstehenden Ansprüche die Steuereinheit veranlassen, ein computerimplementiertes Steuerverfahren auszuführen, mit den Schritten:

- Schalten (S2) der Blutbehandlungsmaschine (1) von einer Hauptschluss-Schaltung, in welcher ein Dialysierflüssigkeitseingang (2.1) und ein Dialysatausgang (2.2) des Dialysators (2) geöffnet sind, sodass frische Dialysierflüssigkeit am Dialysierflüssigkeitseingang (2.1) bereitgestellt wird, Dialysierflüssigkeit durch den Dialysator (2) gefördert wird und verbrauchte Dialysierflüssigkeit bzw. Dialysat am Dialysatausgang (2.2) abgeführt wird, in eine Bypass-Schaltung, in welcher der Dialysierflüssigkeitseingang (2.1) und der Dialysatausgang (2.2) gesperrt sind, und in welcher die frische Dialysierflüssigkeit am Dialysator (2) vorbei geführt wird und ein Dialysierflüssigkeitsfüllvolumen in dem Dialysator (2) eingesperrt wird;
- Erfassen (S3) eines Dialysatausgangswertes (CDO) eines Bestandteils im Dialysat, der mit einem Bluteingangswert (CBI) des Bestandteils in dem Blut an dem Bluteingang (2.3) korreliert, wobei das Erfassen an dem Dialysatausgang (2.2) oder stromabwärts von diesem erfolgt, über eine Erfassungseinheit (32a, 32b, 32c);
- Bereitstellen (S4) eines Signals ($CDO_{pre}$, $CDO_{ext}$) des erfassten Dialysatausgangswertes (CDO) über die Erfassungseinheit (32a, 32b, 32c); und
- Schalten (S5) der Blutbehandlungsmaschine (1) von der Bypass-Schaltung in die Hauptschluss-Schaltung, sodass das eingesperrte Dialysierflüssigkeitsfüllvolumen, von dem insbesondere ein Dialysat-Bolus gebildet ist, am Dialysatausgang (2.2) abgeführt wird;

**gekennzeichnet durch** die Schritte

- Bereitstellen (S1) einer Bypass-Zeit ($t_{BYP}$), für deren Dauer die Blutbehandlungsmaschine (1) in die Bypass-Schaltung geschaltet war, über die Steuereinheit (54);
- Abrufen (S6) eines Kennfeldes eines dialysatorspezifischen Faktors (k), welches in einem Speicher (56), vorzugsweise in der Steuereinheit (54), zumindest in Abhängigkeit der Bypass-Zeit ($t_{BYP}$) abgelegt ist, über die Steuereinheit (54);
- Ermitteln (S7) des dialysatorspezifischen Faktors (k) zumindest in Abhängigkeit der bereitgestellten Bypass-Zeit ($t_{BYP}$) aus dem Kennfeld, über die Steuereinheit (54); und
- Ermitteln (S8) des Bluteingangswertes (CBI) des Bestandteils in dem Blut an dem Bluteingang (2.3) in Abhängigkeit des jeweils zu Beginn der bereitgestellten Bypasszeit ($t_{BYP}$) und nach der bereitgestellten Bypasszeit ($t_{BYP}$) bereitgestellten Signals ($CDO_{pre}$, $CDO_{ext}$) des Dialysatausgangswertes (CDO), sowie des ermittelten, dialysatorspezifischen Faktors (k), über die Steuereinheit (54).

**Claims**

1. Extracorporeal blood treatment machine (1), in particular dialysis machine, for an extracorporeal blood treatment of blood of a patient (P), comprising:

   - a dialyzer (2),
   - an extracorporeal blood circuit (5), which runs through a blood inlet (2.3) and a blood outlet (2.4) of the dialyzer (2) and is adapted to convey blood of the patient (P) through the dialyzer (2),
   - a dialysis fluid circuit (3) with fluidic switching means (26, 34, 40), with a main-connection circuit, in which the dialysis fluid circuit (3) is connected through the dialyzer (2) via a dialysis fluid inlet (2.1) and a dialysate outlet (2.2) of the dialyzer (2) and is adapted to provide fresh dialysis fluid, to convey it through the dialyzer (2) and to discharge used dialysis fluid or dialysate, and a bypass circuit, in which the dialysis fluid circuit (3) is connected past the dialyzer (2) for the duration of a bypass time ($t_{BYP}$) and is adapted to confine a dialysis fluid filling volume (Vo) in the dialyzer (2),
   - a detection unit (32a, 32b, 32c), which is adapted to detect, at the dialysate outlet or downstream of the dialysate outlet (2.2), a dialysate outlet value (CDO) of a constituent in the dialysate, which correlates with a blood inlet value (CBI) of the constituent in the blood at the blood inlet (2.3), and to provide a signal ($CDO_{pre}$, $CDO_{ext}$) of the dialysate outlet value (CDO), and
   - a control unit (54), which is adapted to determine the blood inlet value (CBI) as a function of the signals ($CDO_{pre}$, $CDO_{ext}$) of the dialysate outlet value (CDO) provided at the start of the bypass time ($t_{BYP}$) and after the bypass time ($t_{BYP}$) and of a dialyzer-specific factor (k) dependent on the bypass time ($t_{BYP}$),

   **characterized in that** at least one characteristic curve, preferably a characteristic map, of the dialyzer-specific factor (k) is stored in a memory (56) of the blood treatment machine (1), preferably in the control unit (54), at least as a function of the bypass time ($t_{BYP}$) and can be retrieved to determine the blood inlet value (CBI).

2. Extracorporeal blood treatment machine (1) according to Claim 1, **characterized in that** the at least one characteristic curve, preferably the characteristic map, of the dialyzer-specific factor (k) is stored in the memory (56) of the blood treatment machine (1), preferably in the control unit (54), as a function of a blood flow ($Q_B$) and can be retrieved to determine the blood inlet value (CBI).

3. Extracorporeal blood treatment machine (1) according to Claim 1 or 2, **characterized in that** the control unit (54) is adapted to determine an actual value of at least one characteristic variable (D, K) of the dialyzer (2), in particular of a dialysance (D) and/or of a clearance (K), as a function of the determined constituent in the blood at the blood inlet (CBI).

4. Extracorporeal blood treatment machine (1) according to Claim 3, **characterized in that** the control unit (54) is adapted to estimate a urea distribution volume (V) via a correlation which is stored and can be called up in the memory (56) and to determine a dialysis effectiveness (Kt/V) as a function of this estimated urea distribution volume (V), of the determined characteristic variable (K), in particular of the clearance (K), and of a blood treatment or dialysis duration (t) which is determined or detected, in particular in minutes.

5. Extracorporeal blood treatment machine (1) according to Claim 3 or 4, **characterized in that** a target value of the at least one characteristic variable (D, K), in particular of the dialysance (D) and/or clearance (K), is stored in the memory (56), and the control unit (54) is adapted to continuously determine a deviation of the actual value from the target value of the at least one characteristic variable (D, K).

6.  Extracorporeal blood treatment machine (1) according to Claim 5, **characterized in that** the control unit (54) is adapted to determine, at least as a function of the determined deviation of the at least one characteristic variable (D, K), a fiber change of the dialyzer (2), in particular a secondary membrane formation and/or clotting of fibers of the dialyzer (2), and/or an access recirculation in the extracorporeal blood circuit (5).

7.  Extracorporeal blood treatment machine (1) according to one of the preceding claims, **characterized in that** a pressure detection unit (48) is arranged in the extracorporeal blood circuit (5) upstream of the blood inlet (2.3), which pressure detection unit is adapted to detect a blood inlet pressure and to provide it to the control unit (54), in particular in a time-current manner, and **in that** the control unit (54) is adapted to continuously determine a deviation, in particular an increase, of the provided blood inlet pressure relative to a blood inlet pressure provided at the beginning of the blood treatment.

8.  Extracorporeal blood treatment machine (1) at least according to Claim 5 and 7, **characterized in that** the control unit (54) is adapted to output an indication of a possible fiber change and/or secondary membrane formation in the dialyzer (2) if both the determined deviation of the actual value from the target value of the at least one characteristic variable (D, K) and the determined increase of the provided blood inlet pressure are sufficiently large.

9.  Extracorporeal blood treatment machine (1) at least according to Claim 5 and 7, **characterized in that** the control unit (54) is adapted to output an indication of a possible access recirculation in the extracorporeal blood circuit (5) if only the determined deviation of the actual value from the target value of the at least one characteristic variable (D, K) is sufficiently large.

10. Extracorporeal blood treatment machine (1) according to Claim 9, **characterized in that** the control unit (54) is adapted to quantify the access recirculation (R) by a function of the access recirculation (R) being stored in the memory (56) as a calculation model, in particular as a look-up table or as a typical characteristic curve or typical characteristic map, and being able to be retrieved by the control unit (54).

11. Extracorporeal blood treatment machine (1) according to one of the preceding claims, **characterized in that** the control unit (54) is adapted to determine a signal duration ($t_{sn}$) of the dialysate outlet value (CDO), which extends from an end of the bypass time ($t_{BYP}$) to a time at which the signal of the dialysate outlet value (CDO) again has the dialysate outlet value ($CDO_{pre}$) detected before the bypass time ($t_{BYP}$), and/or **in that** the control unit (54) is adapted to determine a half-value width ($t_{sh}$) of the signal of the dialysate outlet value (CDO) after an end of the bypass time ($t_{BYP}$).

12. Extracorporeal blood treatment machine (1) according to Claim 11, **characterized in that** the control unit (54) is adapted to determine a specific dialysis fluid filling volume (Vo) of the specific dialyzer (2) as a function of the signal duration ($t_{sn}$) or of the half-value width ($t_{sh}$), and of a dialysate flow ($Q_D$).

13. Extracorporeal blood treatment machine (1) according to Claim 12, **characterized in that** the control unit (54) is adapted to determine the specific dialyzer (2) as a function of the determined dialysis fluid filling volume ($V_D$), wherein specific dialyzers with their specific dialysate filling volume (Vo) are stored in the memory (56), in particular in a look-up table stored in the memory (56), and in particular dialyzer-specific target values for the dialysance (D) and/or the clearance (K) are stored.

14. Computer program, comprising commands which, when executed by a control unit of an extracorporeal blood treatment machine according to one of the preceding claims cause the control unit to carry out a computer-implemented control, having the steps:

     - switching (S2) the blood treatment machine (1) from a main-connection circuit, in which a dialysis fluid inlet (2.1) and a dialysate outlet (2.2) of the dialyzer (2) are open, so that fresh dialysis fluid is provided at the dialysis fluid inlet (2.1), dialysis fluid is conveyed through the dialyzer (2) and used dialysis fluid or dialysate is discharged at the dialysate outlet (2.2), into a bypass circuit, in which the dialysis fluid inlet (2.1) and the dialysate outlet (2.2) are blocked, and in which the fresh dialysis fluid is guided past the dialyzer (2) and a dialysis fluid filling volume is confined in the dialyzer (2);
     - detecting (S3) a dialysate outlet value (CDO) of a constituent in the dialysate, which correlates with a blood inlet value (CBI) of the constituent in the blood at the blood inlet (2.3), wherein the detection takes place at the dialysate outlet (2.2) or downstream of the latter, via a detection unit (32a, 32b, 32c);
- providing (S4) a signal (CDO$_{pre}$, CDO$_{ext}$) of the detected dialysate outlet value (CDO) via the detection unit (32a, 32b, 32c); and
- switching (S5) the blood treatment machine (1) from the bypass circuit into the main-connection circuit, so that the confined dialysis fluid filling volume, by which in particular a dialysate bolus is formed, is discharged at the dialysate outlet (2.2); **characterized by** the steps
- providing (S1) a bypass time (t$_{BYP}$), for the duration of which the blood treatment machine (1) was switched into the bypass circuit, via the control unit (54);
- retrieving (S6) a characteristic map of a dialyzer-specific factor (k), which is stored in a memory (56), preferably in the control unit (54), at least as a function of the bypass time (t$_{BYP}$), via the control unit (54);
- determining (S7) the dialyzer-specific factor (k) at least as a function of the provided bypass time (t$_{BYP}$) from the characteristic map, via the control unit (54); and
- determining (S8) the blood inlet value (CBI) of the constituent in the blood at the blood inlet (2.3) as a function of the signal (CDO$_{pre}$, CDOext) of the dialysate outlet value (CDO) respectively provided at the start of the provided bypass time (t$_{BYP}$) and after the provided bypass time (tBYP), and of the determined, dialyzer-specific factor (k), via the control unit (54).

**Revendications**

1. Machine de traitement sanguin extracorporel (1), en particulier machine de dialyse, pour un traitement sanguin extracorporel de sang d'un patient (P), présentant :

    - un dialyseur (2),
    - un circuit sanguin extracorporel (5), lequel traverse le dialyseur (2) par une entrée de sang (2.3) et une sortie de sang (2.4) de celui-ci et est adapté pour acheminer du sang du patient (P) à travers le dialyseur (2),
    - un circuit de liquide de dialyse (3) avec des moyens de commutation fluidique (26, 34, 40), avec un circuit de fermeture principal, dans lequel le circuit de liquide de dialyse (3) est commuté à travers le dialyseur (2) par une entrée de liquide de dialyse (2.1) et une sortie de dialysat (2.2) du dialyseur (2) et est adapté pour fournir du liquide de dialyse frais, l'acheminer à travers le dialyseur (2) et évacuer du liquide de dialyse ou dialysat usagé, et un circuit de dérivation, dans lequel le circuit de liquide de dialyse (3) est dérivé du dialyseur (2) pendant la durée d'un temps de dérivation (t$_{BYP}$) et est adapté pour enfermer un volume de remplissage de liquide de dialyse (V$_D$) dans le dialyseur (2),
    - une unité de détection (32a, 32b, 32c) qui est adaptée pour détecter, à la sortie de dialysat ou en aval de la sortie de dialysat (2.2), une valeur de sortie de dialysat (CDO) d'un composant dans le dialysat qui est corrélé à une valeur d'entrée de sang (CBI) du composant dans le sang à l'entrée de sang (2.3), et pour fournir un signal (CDO$_{pre}$, CDO$_{ext}$) de la valeur de sortie de dialysat (CDO), et
    - une unité de commande (54) qui est adaptée pour déterminer la valeur d'entrée de sang (CBI) en fonction des signaux fournis au début du temps de dérivation (t$_{BYP}$) et après le temps de dérivation (t$_{BYP}$) (CDO$_{pre}$, CDO$_{ext}$) de la valeur de sortie de dialysat (CDO) et d'un facteur spécifique au dialyseur (k) dépendant du temps de dérivation (t$_{BYP}$),

    **caractérisée en ce que**, dans une mémoire (56) de la machine de traitement sanguin (1), de préférence dans l'unité de commande (54), au moins une courbe caractéristique, de préférence un champ caractéristique, du facteur spécifique au dialyseur (k) en fonction au moins du temps de dérivation (t$_{BYP}$) est enregistré et peut être consulté pour déterminer la valeur d'entrée de sang (CBI).

2. Machine de traitement sanguin extracorporel (1) selon la revendication 1, **caractérisée en ce que**, dans la mémoire (56) de la machine de traitement sanguin (1), de préférence dans l'unité de commande (54), au moins une courbe caractéristique, de préférence le champ caractéristique, du facteur spécifique au dialyseur (k) est enregistrée en fonction d'un débit sanguin (Q$_B$) et peut être consultée pour déterminer la valeur d'entrée de sang (CBI).

3. Machine de traitement sanguin extracorporelle (1) selon la revendication 1 ou 2, **caractérisée en ce que** l'unité de commande (54) est adaptée pour déterminer, en fonction du composant déterminé dans le sang à l'entrée de sang (CBI), une valeur réelle d'au moins une grandeur caractéristique (D, K) du dialyseur (2), en particulier une dialysance (D) et/ou une clairance (K).

**4.** Machine de traitement sanguin extracorporel (1) selon la revendication 3, **caractérisée en ce que** l'unité de commande (54) est adaptée pour estimer un volume de distribution d'urée (V) au moyen d'une corrélation enregistrée et pouvant être consultée dans la mémoire (56) et pour calculer, en fonction de ce volume de distribution d'urée estimé (V) estimé, la grandeur caractéristique déterminée (K), en particulier la clairance (K), ainsi qu'une durée de traitement sanguin ou de dialyse (t) déterminée ou enregistrée, en particulier en minutes, pour déterminer une efficacité de dialyse (Kt/V).

**5.** Machine de traitement sanguin extracorporel (1) selon la revendication 3 ou 4, **caractérisée en ce qu'**une valeur de consigne d'au moins une grandeur caractéristique (D, K), en particulier la dialyse (D) et/ou la clairance (K), est enregistrée dans la mémoire (56), et l'unité de commande (54) est adaptée pour déterminer en continu un écart entre la valeur réelle et la valeur de consigne d'au moins une grandeur caractéristique (D, K).

**6.** Machine de traitement sanguin extracorporel (1) selon la revendication 5, **caractérisée en ce que** l'unité de commande (54) est adaptée pour, au moins en fonction de l'écart déterminé de l'au moins une grandeur caractéristique (D, K), détecter une modification des fibres du dialyseur (2), en particulier une formation de membrane secondaire et/ou un colmatage des fibres du dialyseur (2), et/ou une recirculation d'accès dans le circuit sanguin extracorporel (5).

**7.** Machine de traitement sanguin extracorporel (1) selon l'une quelconque des revendications précédentes, **caractérisée en ce qu'**une unité de détection de pression (48) est disposée en amont de l'entrée de sang (2.3) dans le circuit sanguin extracorporel (5), qui est adaptée pour détecter une pression d'entrée de sang et la fournir en continu à l'unité de commande (54), en particulier en temps réel, et **en ce que** l'unité de commande (54) est adaptée pour déterminer en continu un écart, en particulier une augmentation, de la pression d'entrée de sang fournie par rapport à une pression d'entrée de sang fournie au début du traitement sanguin.

**8.** Machine de traitement sanguin extracorporel (1) selon au moins les revendications 5 et 7, **caractérisée en ce que** l'unité de commande (54) est adaptée pour, lorsque à la fois l'écart déterminé entre la valeur réelle et la valeur de consigne d'au moins une grandeur caractéristique (D, K) et l'augmentation déterminée de la pression d'entrée de sang fournie sont suffisamment importantes, émettre une indication d'une possible modification des fibres et/ou d'une formation de membrane secondaire dans le dialyseur (2).

**9.** Machine de traitement sanguin extracorporel (1) selon au moins les revendications 5 et 7, **caractérisée en ce que** l'unité de commande (54) est adaptée pour émettre, lorsque seul l'écart déterminé entre la valeur réelle et la valeur de consigne d'au moins une grandeur caractéristique (D, K) est suffisamment important, une indication d'une possible recirculation d'accès dans le circuit sanguin extracorporel (5).

**10.** Machine de traitement sanguin extracorporel (1) selon la revendication 9, **caractérisée en ce que** l'unité de commande (54) est adaptée pour quantifier la recirculation d'entrée (R) en enregistrant dans la mémoire (56) une fonction de la recirculation d'entrée (R) sous forme d'un modèle de calcul, en particulier sous forme d'un tableau de consultation ou d'une courbe caractéristique ou d'un champ caractéristique, et pouvant être consultée par l'unité de commande (54).

**11.** Machine de traitement sanguin extracorporel (1) selon l'une quelconque des revendications précédentes, **caractérisée en ce que** l'unité de commande (54) est adaptée pour déterminer une durée de signal ($t_{sn}$) de la valeur de sortie de dialysat (CDO) qui s'étend d'une fin du temps de dérivation ($t_{BYP}$) à un moment où le signal de la valeur de sortie de dialysat (CDO) présente à nouveau la valeur de sortie de dialysat ($CDO_{pre}$) enregistrée avant le temps de dérivation ($t_{BYP}$), et/ou **en ce que** l'unité de commande (54) est adaptée pour déterminer, après la fin du temps de dérivation ($t_{BYP}$), une largeur à mi-hauteur ($t_{sh}$) du signal de la valeur de sortie de dialysat (CDO).

**12.** Machine de traitement sanguin extracorporel (1) selon la revendication 11, **caractérisée en ce que** l'unité de commande (54) est adaptée pour déterminer, en fonction de la durée du signal ($t_{sn}$) ou de la largeur à mi-hauteur ($t_{sh}$), ainsi que d'un débit de dialysat ($Q_D$), un volume de remplissage de liquide de dialyse spécifique ($V_D$) du dialyseur spécifique (2).

**13.** Machine de traitement sanguin extracorporel (1) selon la revendication 12, **caractérisée en ce que** l'unité de commande (54) est adaptée pour déterminer le dialyseur spécifique (2) en fonction du volume de remplissage de liquide de dialyse ($V_D$) déterminé, dans laquelle des dialyseurs spécifiques avec leur volume de remplissage de dialyseur spécifique ($V_D$) sont enregistrés dans la mémoire (56), en particulier dans une table de consultation enregistrée dans la mémoire (56), et en particulier des valeurs de consigne spécifiques au dialyseur pour la dialyse (D) et/ou la clairance (K) sont enregistrées.

14. Programme informatique comprenant des instructions qui, lorsqu'elles sont exécutées par l'unité de commande d'une machine de traitement sanguin extracorporel selon l'une quelconque des revendications précédentes, amènent l'unité de commande à exécuter un procédé de commande mis en œuvre par ordinateur, avec les étapes de :

- commutation (S2) de la machine de traitement sanguin (1) d'un circuit de fermeture principal, dans lequel une entrée de liquide de dialyse (2.1) et une sortie de dialysat (2.2) du dialyseur (2) sont ouvertes, de sorte que du liquide de dialyse frais est fourni à l'entrée de liquide de dialyse (2.1), le liquide de dialyse est transporté à travers le dialyseur (2) et le liquide de dialyse usagé ou le dialysat est évacué au niveau de la sortie de dialysat (2.2), dans un circuit de dérivation dans lequel l'entrée de liquide de dialyse (2.1) et la sortie de dialysat (2.2) sont bloquées, et dans lequel le liquide de dialyse frais est acheminé au-delà du dialyseur (2) et un volume de remplissage de liquide de dialyse est bloqué dans le dialyseur (2) ;
- détection (S3) d'une valeur de sortie de dialysat (CDO) d'un composant dans le dialysat, qui est en corrélation avec une valeur d'entrée de sang (CBI) du composant dans le sang à l'entrée de sang (2.3), dans lequel la détection s'effectue à la sortie de dialysat (2.2) ou en aval de celle-ci, par l'intermédiaire d'une unité de détection (32a, 32b, 32c) ;
- fourniture (S4) d'un signal ($CDO_{pre}$, $CDO_{ext}$) de la valeur de sortie de dialysat détectée (CDO) par l'intermédiaire de l'unité de détection (32a, 32b, 32c) ; et
- commutation (S5) de la machine de traitement sanguin (1) du circuit de dérivation au circuit principal, de sorte que le volume de remplissage de liquide de dialyse enfermé, en particulier à partir duquel un bolus de dialysat est formé, est évacué à la sortie de dialysat (2.2) ;

**caractérisé par** les étapes de

- fourniture (51), par l'intermédiaire de l'unité de commande (54), d'un temps de dérivation ($t_{BYP}$) pendant lequel la machine de traitement sanguin (1) a été commutée en circuit de dérivation ;
- consultation (S6), par l'intermédiaire de l'unité de commande (54), d'un champ caractéristique d'un facteur spécifique au dialyseur (k), qui est enregistré dans une mémoire (56), de préférence dans l'unité de commande (54), au moins en fonction du temps de dérivation ($t_{BYP}$) ;
- détermination (S7), par l'intermédiaire de l'unité de commande (54), du facteur spécifique au dialyseur (k) au moins en fonction du temps de dérivation ($t_{BYP}$) fourni à partir du champ caractéristique ; et
- détermination (S8), par l'intermédiaire de l'unité de commande (54), de la valeur d'entrée de sang (CBI) du composant dans le sang à l'entrée de sang (2.3) en fonction du signal fourni respectivement au début du temps de dérivation ($t_{BYP}$) et après le temps de dérivation ($t_{BYP}$) ($CDO_{pre}$, $CDO_{ext}$) de la valeur de sortie de dialysat (CDO), ainsi que du facteur spécifique au dialyseur (k) déterminé.

Fig. 1

EP 4 655 018 B1

Fig. 2

Fig. 3

Fig. 4

Fig. 5

Fig. 6

**IN DER BESCHREIBUNG AUFGEFÜHRTE DOKUMENTE**

**In der Beschreibung aufgeführte Patentdokumente**

- DE 19734992 C1

- DE 102017116097 A1